# EUROPEAN PATENT APPLICATION

(11) **EP 4 609 878 A1**
(43) Date of publication of application: **03.09.2025**
(21) Application number: 23882693.7
(22) Date of filing: 25.10.2023
(51) Int. Cl.: A61K 39/395, A61K 47/02, A61K 47/12, A61K 47/18, A61K 47/20, A61K 47/22, A61K 51/10, A61P 35/00, C07K 1/18

(54) **METHOD FOR PRODUCING RADIOACTIVE PHARMACEUTICAL COMPOSITION**

(30) Priority: 26.10.2022 JP 2022171833
(71) Applicant: Nihon Medi-Physics Co., Ltd., Tokyo 136-0075 (JP)
(72) Inventor: KAWATANI, Minoru, Tokyo 136-0075 (JP); OKUMURA, Yuki, Tokyo 136-0075 (JP); TAKEMORI, Hideaki, Tokyo 136-0075 (JP); TAKENAKA, Fumiaki, Tokyo 136-0075 (JP); ICHIKAWA, Hiroaki, Tokyo 136-0075 (JP)
(74) Representative: TBK
(86) International application number: PCT/JP2023/038582
(87) International publication number: WO 2024/090489

(57) **Abstract**

The present invention relates to a production method of a liquid radioactive pharmaceutical composition containing a radiolabeled antibody, in which the antibody is labeled with the radionuclide, as an active ingredient and one or more additives, including
(Step 1) a step of labeling an antibody with a radionuclide to obtain a solution of a crude product of the radiolabeled antibody;
(Step 2) a step of contacting the solution of the crude product obtained in the Step 1 with a cation exchange material to adsorb the radiolabeled antibody onto the cation exchange material; and
(Step 3) a step of eluting the radiolabeled antibody adsorbed onto the cation exchange material in the Step 2 with an eluent containing the additive.

According to the method of the present invention, a radioactive pharmaceutical composition superior in radiochemical yield and radiochemical purity can be produced by purifying a radiolabeled antibody by a convenient and simple method.

## Description

### [Technical Field]

The present invention relates to a method for producing a radioactive pharmaceutical composition, and a method for purifying a radiolabeled antibody.

### [Background Art]

Conventionally, radionuclides have been used for therapeutic or in vivo diagnostic applications. For example, positron-emitting nuclides and gamma-ray-emitting nuclides are used for tumor diagnosis, and beta-ray-emitting nuclides are used for tumor treatment.

For application of radionuclides to in vivo diagnosis and treatment, the delivery of the radionuclides to the target cell site is required. One method for delivering the radionuclides to the target cell site is to conjugate the radionuclides to biologically useful molecules, such as antibodies.

Non Patent Literature 1 discloses that an anti-IGSF4 antibody that specifically recognizes IgSF4, which is a molecule specifically expressed in liver cancer cells, has been labeled with ⁶⁷Cu. This ⁶⁷Cu-labeled anti-IGSF4 antibody is purified by centrifugation using a centrifugal ultrafiltration filter known under the trade name Amicon after labeling with ⁶⁷Cu.

In addition, Non Patent Literature 2 discloses that an anti-lipoteichoic acid (LTA) antibody, which is an antibody that binds to LTA, has been labeled with ⁸⁹Zr. This ⁸⁹Zr-labeled anti-LTA antibody is also purified by centrifugation using a centrifugal ultrafiltration filter after labeling with ⁸⁹Zr.

As a method for purifying an antibody, Patent Literature 1 discloses purification of rituximab or bevacizumab containing Chinese hamster ovary protein contaminants, using a cation exchange column (POROS 50 HS, Applied Biosystems).

### [Citation List]

### [Patent Literature]

[Patent Literature 1]
   JP 2011-502161 A
[Patent Literature 2]
   JP 2010-270068 A

### [Non Patent Literature]

[Non Patent Literature 1]
   Katsumasa Fujiki, et al., Scientific Reports, 2017, 7, 1912.
[Non Patent Literature 2]
   Julie E. Pickett, et al., Bone Research, 2018, 6, 13.

### [Summary of Invention]

A characteristic of radionuclides used for therapeutic or in vivo diagnostic purposes is that they have a short half-life, and decay and disappear in a short period of time. For this reason, it is demanded to manufacture radioactive pharmaceutical compositions in a shorter period of time.

In addition, a method that reduces radioactive contamination of equipment and radiation exposure of workers as much as possible is required in the production of radioactive pharmaceutical compositions. On the other hand, molecules labeled with radionuclides that become the active ingredient only need to have radioactivity in an amount that can demonstrate therapeutic or in vivo diagnostic use, and a sufficient mass of substance is generally small. For this reason, in the case of radio-labeling low-molecular-weight substances, a series of processes from synthesis to purification has been automated using a small device such as that shown in Patent Literature 2. However, there are still various problems in automating the manufacturing of radiolabeled antibodies.

The purification methods using a centrifuge shown in Non Patent Literatures 1 and 2 are difficult to automate.

Patent Literature 2 does not disclose automating the synthesis and purification of radiolabeled antibodies.

Patent Literature 1 aims at improving the removal of Chinese hamster ovary protein contaminants, and does not take note of the problems relating to the manufacturing of radiolabeled antibodies.

The present invention aims to manufacturing a radioactive pharmaceutical composition superior in radiochemical yield and radiochemical purity by purifying a radiolabeled antibody by a convenient and simple method.

The present inventors have conducted intensive studies in view of the above-mentioned problems and succeeded in contacting a solution of a crude product of a radiolabeled antibody with a cation exchange material to adsorb the radiolabeled antibody onto the cation exchange material, and then eluting the adsorbed radiolabeled antibody by using an eluent containing one or more additives, and completed the present invention.

One embodiment of the present invention is a method for manufacturing a liquid radioactive pharmaceutical composition containing, as an active ingredient, a radiolabeled antibody obtained by labeling an antibody with a radionuclide, and containing one or more additives (A), comprising:
(Step 1) a step of labeling an antibody with a radionuclide to obtain a solution of a crude product of the aforementioned radioactive labeled antibody;
(Step 2) a step of contacting the solution of the crude product obtained in the aforementioned Step 1 with a cation exchange material to adsorb the aforementioned radiolabeled antibody onto the aforementioned cation exchange material; and
(Step 3) a step of eluting the aforementioned radiolabeled antibody adsorbed onto the aforementioned cation exchange material in the aforementioned Step 2 with an eluent containing the aforementioned additive (A).

Another embodiment of the present invention is a method for purifying a radiolabeled antibody, comprising:
(Step A) a step of labeling an antibody with a radionuclide to obtain a solution of a crude product of a radiolabeled antibody;
(Step B) a step of contacting the solution of the crude product obtained in the aforementioned Step A with a cation exchange material to adsorb the aforementioned radiolabeled antibody onto the aforementioned cation exchange material; and
(Step C) a step of eluting the aforementioned radiolabeled antibody adsorbed onto the cation exchange material in the aforementioned Step B with an eluent containing one or more additives.

According to the present invention, since the radiolabeled antibody is purified using a cation exchange material, the radiolabeled antibody can be easily purified and can also be applied to an automated synthesizer. Furthermore, since a part of the formulation is used as an eluent, the composition manufacturing process can be simplified and a radioactive pharmaceutical composition can be prepared in a shorter time. Therefore, according to the present invention, it is possible to manufacture a radioactive pharmaceutical composition superior in radiochemical yield and radiochemical purity.

### [Description of Embodiments]

Unless otherwise specified, the terms used in the present specification can be used with the meanings generally used in the pertinent technique field.

### (1) Pharmaceutical composition and manufacturing method thereof

The present invention provides a liquid radioactive pharmaceutical composition (hereinafter also to be referred to as the liquid pharmaceutical composition of the present invention) containing, as an active ingredient, an antibody labeled with a radionuclide (hereinafter also to be referred to as the radiolabeled antibody of the present invention).

The present invention also provides a method for producing a liquid radioactive pharmaceutical composition containing a radiolabeled antibody as an active ingredient, and containing one or more additives (A), including
(Step 1) a step of labeling an antibody with a radionuclide to obtain a solution of a crude product of the radiolabeled antibody;
(Step 2) a step of contacting the solution of the crude product obtained in Step 1 with a cation exchange material to adsorb the radio-labeled antibody onto the cation exchange material; and
(Step 3) a step of eluting the radiolabeled antibody adsorbed onto the cation exchange material in Step 2 with an eluent containing one or more additives.

In the radiolabeled antibody of the present invention, a radionuclide and an antibody form a conjugate (hereinafter also to be referred to as the conjugate of radionuclide and antibody of the present invention). In the conjugate of radionuclide and antibody of the present invention, antibody and radionuclide may be directly connected. In addition, in the conjugate of radionuclide and antibody of the present invention, antibody and radionuclide may be connected via a linker. In this case, the radionuclide may be a radioactive metal nuclide, and the radioactive metal nuclide may form a chelate (complex) with the chelating agent, and the chelating agent may be connected to the antibody via or not via a linker. The connection here may preferably be connection by a covalent bond. Each step is described in the following.

### Step 1: Step of labeling an antibody with a radionuclide to obtain a solution of a crude product of the radiolabeled antibody

### (1-1) Radionuclide

The radionuclide contained in the radiolabeled antibody of the present invention may be a radionuclide that emits α particles, positron, β-ray, or γ-ray, or a radioactive halogen nuclide. Examples of the radionuclide that emits α particles include ²¹¹At, ²¹²Bi, ²¹³Bi, ²²⁵Ac, ²²⁷Th. Examples of the radionuclide that emits positron include ⁶⁴Cu, ⁶⁸Ga, ⁸⁶Y, ⁸⁹Zr. Examples of the radionuclide that emits β-ray include ⁶⁴Cu, ⁹⁰Y or ¹⁷⁷Lu. Examples of the radionuclide that emits γ-ray include ^{99m}Tc or ¹¹¹In. Examples of the radioactive halogen nuclide include ¹⁸F, Al¹⁸F, ⁷⁷Br, ¹²³I, ¹²⁵I, ¹³¹I or ²¹¹At. The radionuclide contained in the RI-labeled antibody of the present invention, and thus in the liquid pharmaceutical composition of the present invention, is preferably Al¹⁸F, ⁸⁹Zr, ⁹⁰Y, ¹¹¹In, ¹⁷⁷Lu, or ²²⁵Ac.

These radionuclides can be produced by a predetermined nuclear reaction, or can be obtained as commercial products available from Eckert & Ziegler, Thermo Fisher Scientific, Institute of Isotopes, POLATOM, and the like. The radionuclides thus produced or obtained can be used to form a conjugate with an antibody by subjecting them to a chemical treatment such as chelating to make them into a chemical form suitable for binding to an antibody, whereby the antibody is labeled with the radionuclide.

### (1-2) Antibody

The antibody contained in the liquid pharmaceutical composition of the present invention and the radiolabeled antibody of the present invention is not particularly limited, and includes monoclonal antibodies (including full-length monoclonal antibodies), polyclonal antibodies, multispecific antibodies (e.g., bispecific antibodies), and antibody fragments thereof as long as they exhibit the desired binding specificity.

These antibodies are generally expected to afford a therapeutic effect when administered to a mammal suffering from a disease or disorder, and examples thereof include antibodies against CD polypeptides such as CD3, CD4, CD8, CD19, CD20, and CD34; members of the HER receptor family such as EGF receptor (HER1), HER2, HER3, or HER4 receptor; cell adhesion molecules such as LFA-1, Mac1, p150, 95, VLA-4, ICAM-1, VCAM, and av/b3 integrin, including either the a or b subunit (e.g., anti-CD11a, anti-CD18, or anti-CD11b antibody); growth factors such as VEGF; IgE; blood group antigens; flk2/flt3 receptors; obesity (OB) receptors; mpl receptors; CTLA-4; and protein C.

Specific examples of antibodies to be purified include, but are not limited to, the antibodies described in Table 1.

**[Table 1-1]**

| Classification | general name |
|---|---|
| humanized anti-HER2 antibody | trastuzumab |
| chimeric anti-CD20 antibody | rituximab |
| humanized anti-RS virus antibody | palivizumab |
| chimeric anti-TNFα antibody | infliximab |
| chimeric anti-CD25 antibody | basiliximab |
| humanized anti-IL6R antibody | tocilizumab |
| humanized anti-VEGF antibody | bevacizumab |
| human anti-TNFα antibody | adalimumab |
| chimeric anti-EGFR antibody | cetuximab |
| humanized anti-VEGF antibody fragment | ranibizumab |
| humanized anti-IgE antibody | omalizumab |
| humanized anti-complement C5 antibody | eculizumab |
| human anti-EGFR antibody | panitumumab |
| human anti-IL12/IL23-p40 antibody | ustekinumab |
| human anti-TNFα antibody | golimumab |
| human anti-IL-1β antibody | canakinumab |
| human anti-RANKL antibody | denosumab |
| humanized anti-CCR4 antibody | mogamulizumab |
| PEG-modified humanized anti-TNF antibody fragment | certolizumab |
| human anti-CD20 antibody | ofatumumab |
| humanized anti-HER2 antibody | pertuzumab |
| humanized anti-α4 integrin antibody | natalizumab |
| human anti-PD-1 antibody | nivolumab |
| humanized anti-CD52 antibody | alemtuzumab |
| human anti-IL-17A antibody | secukinumab |
| human anti-VEGFR-2 antibody | ramucirumab |
| humanized anti-CTLA-4 antibody | ipilimumab |
| human anti-PCSK9 antibody | evolocumab |
| humanized anti-IL-5 antibody | mepolizumab |
| human anti-PCSK9 antibody | alirocumab |
| humanized anti-IL-17 antibody | ixekizumab |
| humanized anti-IL-17R antibody | brodalumab |
| humanized anti-dabigatran antibody | idarucizumab |
| humanized anti-SLAMF7 antibody | elotuzumab |
| humanized anti-PD-1 antibody | pembrolizumab |
| human anti-IL-6 receptor α subunit antibody | sarilumab |
| human anti-Clostridium difficile toxin B antibody | bezlotoxumab |
| human anti-BlyS antibody | belimumab |

**[Table 1-2]**

| | |
|---|---|
| human anti-CD38 antibody | daratumumab |
| human anti-CD38 antibody + human hyaluronidase analog | daratumumab+ hyaluronidase alpha |
| human anti-IL-4Rα subunit antibody | dupilumab |
| humanized anti-PD-L1 antibody | atezolizumab |
| humanized anti-IL-5Rα subunit antibody | benralizumab |
| humanized anti-F.IXa/anti-F.X bispecific antibody | emicizumab |
| human anti-IL-23 antibody | guselkumab |
| human anti-PD-L1 antibody | durvalumab |
| humanized anti-CD20 antibody (glyco-modified) | obinutuzumab |
| humanized anti-α4β7 integrin antibody | vedolizumab |
| Mouse anti-CD19/anti-CD3 bispecific antibody (scFv-scFv) | blinatumomab |
| humanized anti-sclerostin antibody | romosozumab |
| humanized anti-IL-23α (p19) subunit antibody | risankizumab |
| human anti-EGFR antibody | necitumumab |
| humanized anti-human complement C5 antibody | ravulizumab |
| human anti-human FGF23 antibody | burosumab |
| humanized human VEGF antibody (scFv) | brolucizumab |
| chimeric anti-CD38 antibody | isatuximab |
| humanized anti-IL-23α (p19) subunit antibody | tildrakizumab |
| humanized anti-IL-6 receptor antibody | satralizumab |
| humanized anti-CGRP antibody | galcanezumab |
| humanized anti-CD19 antibody | inebilizumab |
| humanized anti-CGRP antibody | fremanezumab |
| human anti-CGRP type 1 receptor antibody | erenumab |
| chimeric anti-GD2 antibody | dinutuximab |
| human anti-SARS-CoV-2 spike protein antibody | casirivimab+i mdevimab |
| human anti-type I interferon receptor 1 antibody | anifrolumab |
| human anti-SARS-CoV-2 spike protein antibody | sotrovimab |
| humanized anti-IL-17A/IL17-F antibody | bimekizumab |
| anti-VEGF-A/anti-Ang2 bispecific antibody | faricimab |
| humanized anti-IL-31 receptor antibody | nemolizumab |
| human anti-plasma kallikrein antibody | lanadelumab |

The antibody may also be conjugated with a drug, and several antibody-drug conjugates have been approved.

Preferred examples include trastuzumab, pertuzumab, panitumumab, rituximab, and bevacizumab.

In the present specification, when the antibody is radiolabeled, it is indicated as "radioactive" for distinction.

### (1-3) Chelating agent

Step 1 of the present invention is a step of labeling an antibody with a radionuclide to obtain a solution of a crude product of a radiolabeled antibody to which radionuclide labeled.

The method for labeling an antibody with a radionuclide is not particularly limited, and a chelating agent is preferably used. The chelating agent is not particularly limited as long as it has a site in the structure thereof where radionuclide is coordinated. Preferably, it has a chelating moiety, which is a site to which a radionuclide is coordinated, and a substituent that enables conjugating with an antibody. Examples of the chelating moiety include CB-TE2A (1,4,8,11-Tetraazabicyclo[6.6.2]hexadecane-4,11-diacetic acid), CDTA (Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-α,α',α",α‴-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-Carboxyethyl)-1,4,7,10-tetraazacyclododecane-1,4,7,10-tetraacetic acid), DOTA-GA-NHS, DOTP (((1,4,7,10-Tetraazacyclododecane-1,4,7,10-tetrayl)tetrakis(methylene))tetraphosphonic acid), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane(L^{py}), p-SCN-Bn-DOTA (S-2-(4-Isothiocyanatobenzyl)-1,4,7,10-tetraazacyclododecane tetraacetic acid), MeO-DOTA-NCS (1-[(2-methoxy-5-isothiocyanatophenyl)-carboxymethyl]-4,7,10-triscarboxy 5 methyl-1,4,7,10-tetraazacyclododecane), EuK-106, DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), DTPA-BMA (5,8-Bis(carboxymethyl)-11-[2-(methylamino)-2-oxoethyl]-3-oxo-2,5,8,11-tetraazatridecan-13-oic acid), EDTA (2,2',2",2‴-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid), TETPA (1,4,8,11-tetraazacyclotetradecane-1,4,8,11-tetrapropionic acid), TETA (1,4,8,11-Tetraazacyclotetradecane-N,N',N",N'"-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N",N'"-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N",N'",N""-penta-acetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridine-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N"-bis(6-carboxy-2-pyridylmethyl)-diethylenetriamine-N,N',N"-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), porphyrin, DO3A (1,4,7,10-Tetraazacyclododecane-1,4,7-triacetic acid trisodium salt), and DO3A-NHS (1,4,7,10-Tetraazacyclododecane-1,4,7,10-tetraacetic acid mono-N-hydroxysuccinimide ester). It is preferable to have a structure derived from a compound represented by the following formula (A).

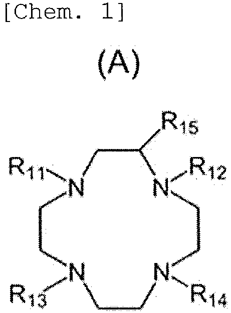

In the formula (A), R₁₁, R₁₃ and R₁₄ are each independently a group consisting of -(CH₂)ₚCOOH, -(CH₂)ₚC₅H₄N, - (CH₂)ₚPO₃H₂, - (CH₂)ₚCONH₂ or -(CHCOOH) (CH₂)ₚCOOH, one of R₁₂ and R₁₅ is a hydrogen atom, a carboxyl group, or a carboxyalkyl group having 2 or 3 carbon atoms, the other is a substituent for conjugating with the aforementioned antibody, p is an integer of not less than 0 and not more than 3, R₁₅ is a hydrogen atom when R₁₂ is a substituent for conjugating with the aforementioned antibody, and R₁₅ is a substituent for conjugating with the aforementioned antibody when R₁₂ is not a substituent for conjugating with the aforementioned antibody.

Examples of the specific structure represented by the formula (A) include structures derived from the compounds represented by the following formulas (A-1) to (A-13).

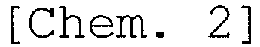

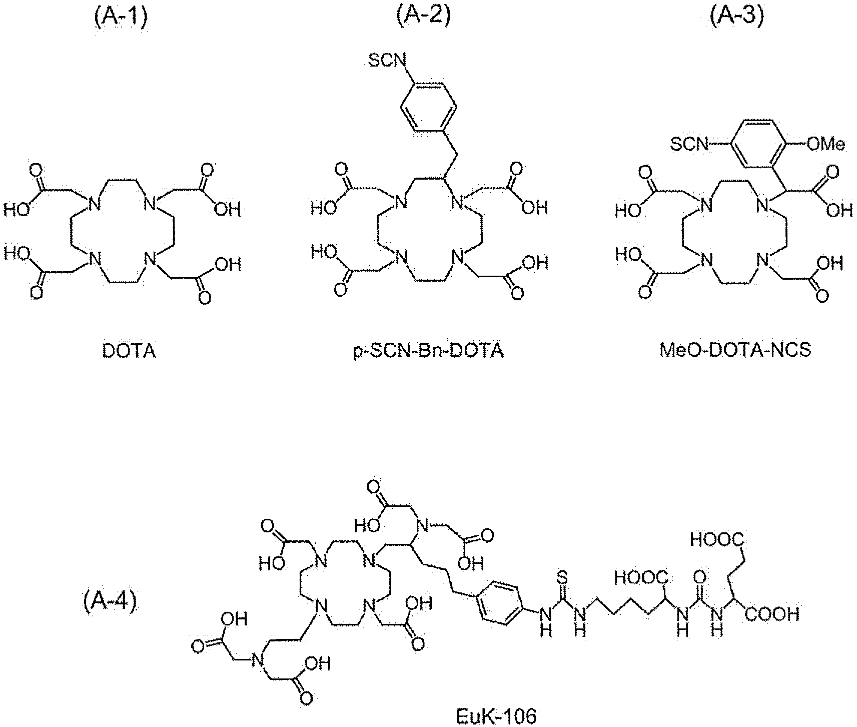

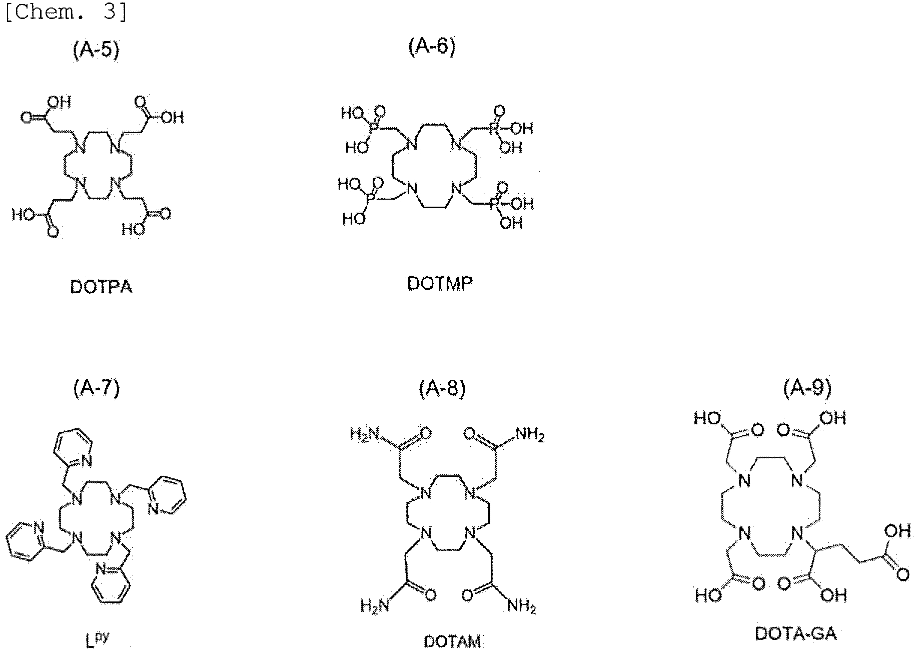

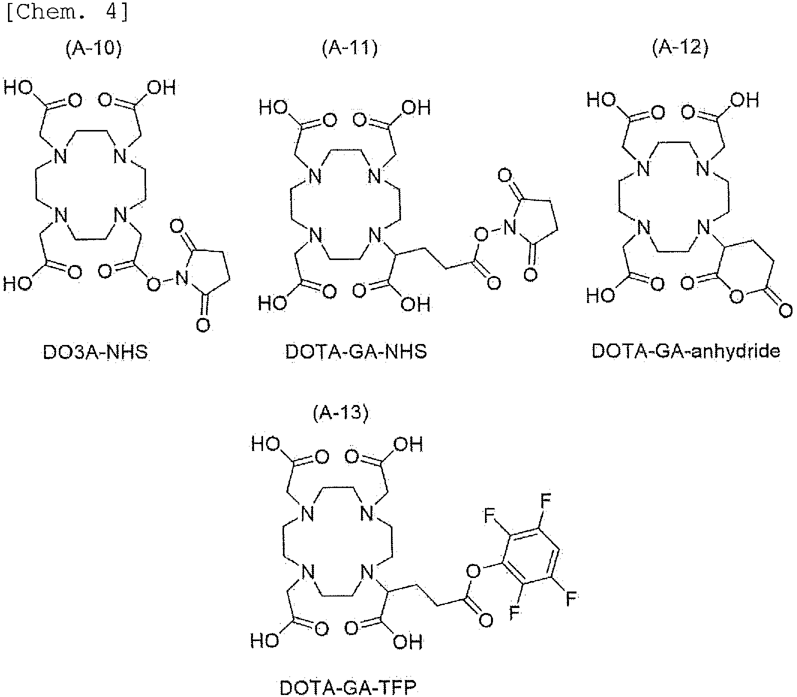

The linkage site between the chelating moiety and the substituent that enables formation of a conjugate with the antibody is preferably an amide bond or a thiourea bond, more preferably an amide bond from the aspect of stability.

The amide bond can be formed, for example, by the reaction of an N-hydroxysuccinimide ester (NHS) group of the above-mentioned formula (A-10) or (A-11), or a 2,6-dioxo tetrahydro-2H-pyranyl group of the above-mentioned (A-12), or a 2,3,5,6-tetrafluorophenol ester group of the above-mentioned (A-13) with primary amine. The thiourea bond can be formed by the reaction of an isothiocyanate group of the compound of the above-mentioned formula (A-2), (A-3) with primary amine.

In the radiolabeled antibody of the present invention, the chelating agent may be provided at least not less than one molecule, preferably not less than 1 molecule and not more than 8 molecules, per one molecule of the antibody. To maintain the activity of the antibody itself (antigen recognition action, neutralizing action, complement activating action and/or opsonin action), a chelating agent is preferably introduced site-specifically into the Fc region (constant region) of the antibody. In the present invention, the chelating agent is preferably provided at one or two molecules per one molecule of the antibody. The chelating agent is more preferably provided at one molecule per one molecule of the antibody.

In the radiolabeled antibody of the present invention, the chelating agent may be connected to the antibody via a linker. Examples of the linker include substituted or unsubstituted alkyl group, substituted or unsubstituted heteroalkyl group, polyethylene glycol (PEG) group, peptides, sugar chain, disulfide group, combination of these and the like.

Preferably, the chelating agent modifies the antibody site-specifically, more preferably in the Fc region, via a linker. In this case, the linker contains a peptide consisting of not less than 13 and not more than 17 amino acid residues represented by the following formula (i) (hereinafter, to be also referred to as "antibody-modification peptide"), and one formed by a cross-linking reaction between the antibody-modification peptide modified with a crosslinking agent and the antibody can be used. In the explanation of the formula (i), the left side of the amino acid sequence on the page indicates the N-terminal side, and the right side of the amino acid sequence on the page indicates the C-terminal side. When the chelating agent is connected to the antibody via the antibody-modification peptide as a linker, the position where the chelating agent and the antibody-modification peptide are linked is not particularly limited. For example, it can be directly or indirectly linked at the N-terminal or C-terminal of the antibody-modification peptide, preferably at the N-terminal. In addition, the C-terminal of the antibody-modification peptide may be modified by, for example, amidation or the like to improve its stability and the like.

(Xa)-Xaa₁-(Xb)-Xaa₂-(Xc)-Xaa₃-(Xd) (i)

In the formula (i), Xa, Xb, Xc and Xd are each continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa₁ and Xaa₃ are each independently an amino acid residue derived from an amino acid having a thiol group in the side chain, or
one is an amino acid residue derived from an amino acid having a thiol group in the side chain and the other is an amino acid residue derived from an amino acid having a haloacetyl group in the side chain, and Xaa₁ and Xaa₃ are linked, and
Xaa₂ is a lysine residue, arginine residue, cysteine residue, aspartic acid residue, glutamic acid residue, 2-aminosuberic acid, or diamino propionic acid, and modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

In the formula (i), a, b, c and d are not particularly limited as long as they are numbers within the aforementioned range. From the aspect of the stability of binding between the peptide and antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

Xaa₁ and Xaa₃ are amino acid residues derived from an amino acid having a thiol group in the side chain, and Xaa₁ and Xaa₃ may be the same or different. Examples of the amino acid having a thiol group in the side chain include cysteine and homocysteine. Such amino acid residues are preferably bonded by a disulfide bond, or a sulfide group is preferably bonded thereto via a linker shown by the following formula (4). In the formula (4), the wavy line indicates the binding part with the sulfide group.

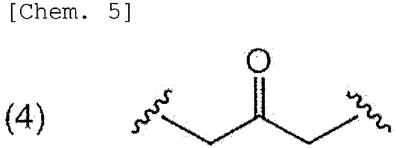

Instead of the aforementioned combination of Xaa₁ and Xaa₃, one of Xaa₁ and Xaa₃ may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Among these, the amino acid sequence of the antibody-modification peptide preferably has any one of the following sequences (1) to (14), more preferably the following sequence (1), (2), (13) or (14). In the following amino acid sequences (1) to (14), (Xaa₂) is a lysine residue, a cysteine residue, an aspartic acid residue, a glutamic acid residue, a 2-aminosuberic acid, or a diamino propionic acid, and (Xaa₁) and (Xaa₃) are each a homocysteine residue. In the following amino acid sequences (1) to (14), the amino acids other than (Xaa₁), (Xaa₂) and (Xaa₃) are indicated by one-letter abbreviations.

(1) DCAYH(Xaa₂)GELVWCT (SEQ ID NO: 1)
(2) GPDCAYH(Xaa₂)GELVWCTFH (SEQ ID NO: 2)
(3) RCAYH(Xaa₂)GELVWCS (SEQ ID NO: 3)
(4) GPRCAYH(Xaa₂)GELVWCSFH (SEQ ID NO: 4)
(5) SPDCAYH(Xaa₂)GELVWCTFH (SEQ ID NO: 5)
(6) GDDCAYH(Xaa₂)GELVWCTFH (SEQ ID NO: 6)
(7) GPSCAYH(Xaa₂)GELVWCTFH (SEQ ID NO: 7)
(8) GPDCAYH(Xaa₂)GELVWCSFH (SEQ ID NO: 8)
(9) GPDCAYH(Xaa₂) GELVWCTHH (SEQ ID NO: 9)
(10) GPDCAYH(Xaa₂)GELVWCTFY (SEQ ID NO: 10)
(11) SPDCAYH(Xaa₂)GELVWCTFY (SEQ ID NO: 11)
(12) SDDCAYH(Xaa₂)GELVWCTFY (SEQ ID NO: 12)
(13) RGNCAYH(Xaa₂)GQLVWCTYH (SEQ ID NO: 13)
(14) G(Xaa₁)DCAYH(Xaa₂)GELVWCT(Xaa₃) H (SEQ ID NO: 14)

### (1-4) Production method of radiolabeled antibody

The production method of the radiolabeled antibody of the present invention is described.

In the radiolabeled antibody of the present invention, the conjugate of radionuclide and antibody of the present invention may be prepared, for example, by introducing radioactive iodine (¹²³I, ¹³¹I) as a radionuclide into the tyrosine residue of the antibody. Alternatively, the conjugate may be prepared by introducing a substituent to which a radioactive halogen nuclide stably binds, into the antibody of the present invention and reacting the resulting antibody with a radioactive halogen ion.

The radiolabeled antibody of the present invention can be produced by two steps of a conjugation step of conjugating a chelating agent and an antibody, and a complex formation step of forming a complex of a radionuclide and a chelating agent (production step of chelating agent with radionuclide chelated). The conjugation step may be performed before the complex formation step or after the complex formation step.

In the conjugation step, various methods for chemical modification of antibody are used. Specifically, the methods (a) - (f) can be mentioned:
(a) amine coupling method (a method for modifying the amino group of a lysine residue of an antibody by using a chelating agent or chelate having a carboxyl group activated by an N-hydroxysuccimidyl (NHS) group)
(b) method for modifying a sulfhydryl (SH) group generated by partially reducing a disulfide bond (SS bond) between polypeptide chains at the hinge site of an antibody with a chelating agent or linker having a maleimide group reactive with the SH group
(c) method for modifying cysteine newly introduced into an antibody by an amino acid mutation by genetic engineering with a chelating agent or linker having a maleimide group
(d) method for modifying an azide group of lysine azide newly introduced into an antibody by an amino acid mutation by genetic engineering with a chelating agent or linker having alkyne (e.g., Dibenzocyclooctyne: DBCO) by using a click reaction
(e) method for modifying glutamine introduced into a specific position of an antibody with a chelating agent or linker having a side chain of lysine by using transglutaminase
(f) method for site-specifically modifying the Fc region of an antibody with a chelating agent or linker having the antibody-modification peptide shown in the aforementioned (i)

In the complex formation step, the chelating agent is chelated with a radionuclide (complex formation). The radionuclide used here is preferably used in a manner permitting ionization, more preferably in the form of an ion, from the viewpoint of increasing the complex formation efficiency. In the complex formation step, the order of addition of the radionuclide to the chelating agent does not matter as long as a complex of a radionuclide can be formed. For example, a solution in which radionuclide ions are dissolved in a solvent mainly composed of water can be used as a radionuclide.

After complex formation, the obtained complex may be purified using a filtration filter, a membrane filter, a column filled with various fillers, chromatography or the like.

In the production method of the radiolabeled antibody of the present invention, a conjugation step is preferably performed after the complex formation step.

In a more preferred embodiment, in complex formation step (A), a complex is formed between a radionuclide and a chelating agent having a first atomic group capable of click reaction as a substituent for enabling conjugation with the antibody. Then, in conjugation step (B), using an antibody-modification peptide shown by the aforementioned (i) and an antibody-modification linker having a second atomic group capable of click reaction, a click reaction is performed between the peptide-modified antibody in which Fc region is site-specifically modified and the chelating agent with a formed complex which is obtained in step (A) to obtain the chelate conjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker is preferably alkyne and the antibody-modification linker is preferably azide, or the chelate linker is preferably 1,2,4,5-tetrazine and the antibody-modification linker is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzocyclooctyne (DBCO) as alkyne of the first atomic group (the formula (1a)) and an atomic group containing an azide group as azide of the second atomic group (the formula (2a)), or a combination of an atomic group containing 1,2,4,5-tetrazine as the first atomic group (the formula (1b)) and an atomic group containing trans-cyclooctene (TCO) as alkene of the second atomic group (the formula (2b)). Preferred is the combination of the formula (1a) and the formula (2a). 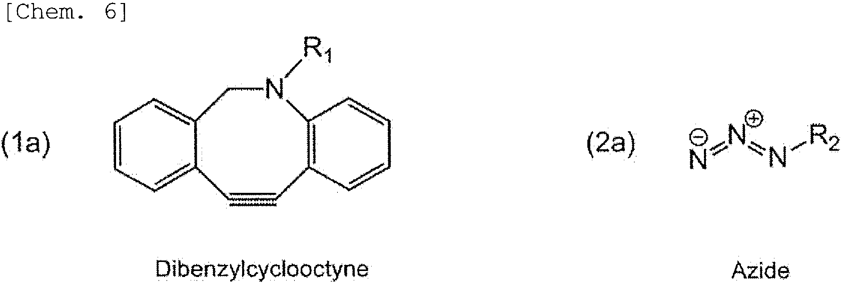 wherein R₁ is a linkage site with a chelating agent, and R₂ is a linkage site with an antibody-modification peptide in the antibody. 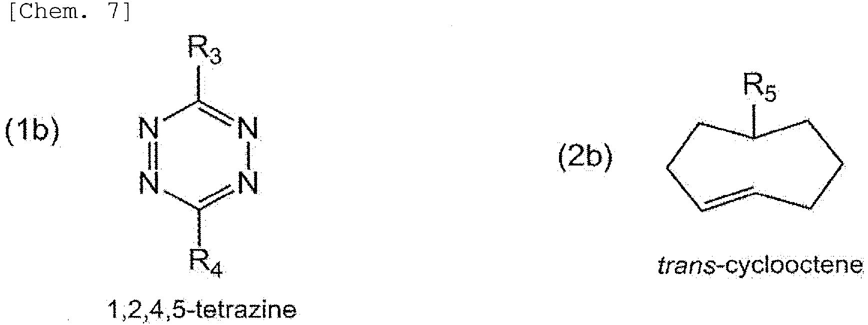 wherein one of R₃ and R₄ is a linkage site with a chelating agent or an antibody-modification peptide in the antibody, and the other is a hydrogen atom, a methyl group, a phenyl group or a pyridyl group, and R₅ is a linkage site with any one chelating agent or an antibody-modification peptide in the antibody depending on R₃ or R₄.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzocyclooctyne-Amine, Dibenzocyclooctyne Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzocyclooctyne Maleimide is preferably used.

In step (A), more preferably, a chelating agent having a structure represented by the following formula (ii) is used.

A-B-C (ii)

In the formula (ii), A is a chelating moiety represented by the following formula (iia).

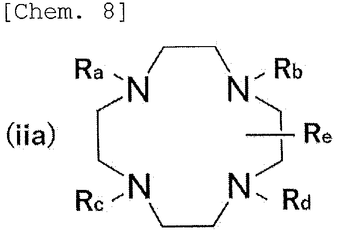

In the formula (iia), Ra, Rb, and Rc are each independently a group consisting of -(CH₂)ₚCOOH, -(CH₂)ₚC₅H₄N, - (CH₂)ₚPO₃H₂, -(CH₂)ₚCONH₂, or - (CHCOOH) (CH₂)ₚCOOH, p is an integer of not less than 0 and not more than 3, either Rd or Re is a binding site (*) to B, and the other is a hydrogen atom or a group consisting of -(CH₂)ₚCOOH, -(CH₂)ₚC₅H₄N, -(CH₂)ₚPO₃H₂, - (CH₂)ₚCONH₂, or -(CHCOOH) (CH₂)ₚCOOH, and p is an integer of not less than 0 and not more than 3.

In the formula (ii), B is represented by the following formula (iib):

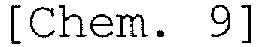

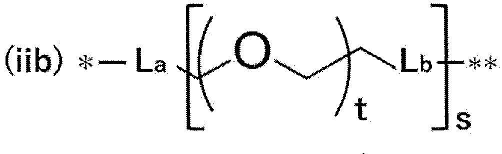

In the formula (iib), La and Lb are each independently a bond linker containing at least an amide bond or thiourea bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

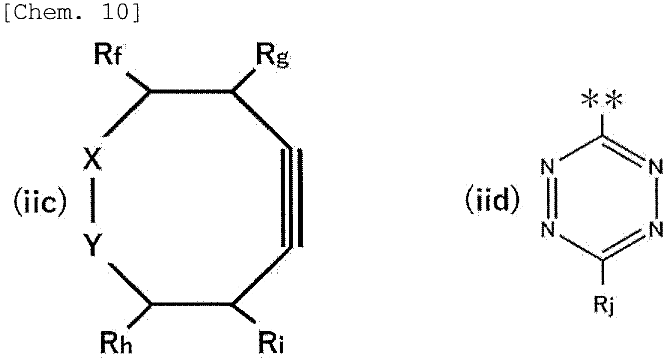

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbocycle, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (iia) wherein Ra to Rd are -(CH₂)ₚCOOH, p is 1, Re is a binding site with B; or DO3A derivative or DOTA-GA derivative wherein Ra to Rc are - (CH₂)ₚCOOH, p is 1, Rd is a binding site with B, and Re is a hydrogen atom is more preferred.

In the formula (ii), a DOTA-PEGt-DBCO derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B; or a DOTA-PEGt-Tz derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is a tetrazine derivative represented by the formula (iid) is further preferred.

In the formula (ii), a DO3A-PEGt-DBCO derivative wherein A is the above-mentioned DO3A derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the formula (ii), a DOTA-GA-PEGt-DBCO derivative wherein A is the above-mentioned DOTA-GA derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the molar ratio of the chelating agent and radionuclide, as chelating moiety/radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 µmol/L, more preferably not less than 0.01 µmol/L, further preferably not less than 0.1 µmol/L, more preferably not less than 1 µmol/L, and the upper limit is preferably not more than 1000 µmol/L, more preferably not more than 100 µmol/L, further preferably not more than 10 µmol/L. For example, it is within the range of not less than 1 µmol/L and not more than 100 µmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

In the reaction time, provided that the reaction temperature is as described above, lower limit is preferably not less than 5 minutes, more preferably not less than 10 minutes, further preferably not less than 20 minutes, further more preferably not less than 30 minutes, particularly preferably not less than 45 minutes, and the upper limit is preferably not more than 180 minutes, more preferably not more than 150 minutes, further preferably not more than 120 minutes, further more preferably not more than 90 minutes, and especially not more than 60 minutes, for example, and a range of not less than 10 minutes and not more than 150 minutes is preferred, and not less than 10 minutes and not more than 60 minutes is more preferred.

The antibody to be used in step (B) is a peptide-modified antibody in which Fc region (constant region) of humanized antibody as described in detail in the above-mentioned "(1-2) Antibody" is site-specifically modified using the antibody-modification peptide shown in the aforementioned (i), and an antibody-modification linker having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker may be one in which an antibody-modification peptide and a linker represented by the following formula (S1) are bonded.

*-((L₁)ₘ-Z)ₖ-L₂-AG₂ (S1)

wherein * is a binding site with the N-terminal or C-terminal of peptide,
L₁ is a linker moiety of polyethylene glycol (PEG),
m is an integer of not less than 1 and not more than 50,
Z is a second linker moiety that binds (L₁)ₘ and L₂,
k is 0 or 1,
L₂ is the second PEG linker moiety, and
AG₂ is a second atomic group.

In the aforementioned formula (S1), the structure of Z is not particularly limited as long as it is a linker structure that binds (L₁)ₘ and L₂ to each other, and includes, for example, an amino acid sequence consisting of not less than 1 and not more than 5 amino acid residues. In this case, the amino acid sequence contained in Z preferably contains a cysteine residue, and is more preferably bonded to L₂ via a thioether group formed by the bond between the thiol group of the cysteine residue and a maleimide group.

In the present invention, the PEG linker moiety constituting L₂ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

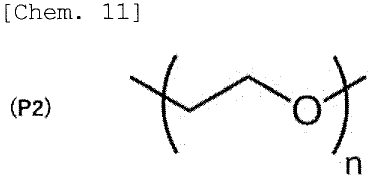

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker, an introduction method including obtaining an antibody-modification peptide having a desired amino acid sequence by the aforementioned method, dissolving the peptide in a solution containing a solubilizing agent and a reducing agent and where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to an antibody to obtain a peptide-modified antibody can be performed, for example, using a crosslinking agent. A crosslinking agent is a chemical substance for linking an antibody-modification peptide and an antibody by a covalent bond. Examples thereof include a crosslinking agent preferably containing two or more succinimidyl groups such as disuccinimidyl glutarate (DSG), disuccinimidyl suberate (DSS) and the like, a crosslinking agent consisting of a compound containing two or more imidic acid moieties such as dimethyl adipimidate and the like, or a salt thereof, a crosslinking agent consisting of a compound having a disulfide bond such as dimethyl 3,3'-dithiobispropionimidate, dithiobissuccinimidylpropionic acid, and the like, or a salt thereof, and the like. Using such crosslinking agent, a crosslinking reaction can be caused between an amino acid residue of Xaa2 in the antibody-modification peptide and an antibody. When, for example, the humanized antibody of the present invention is used as the antibody, the crosslinking reaction in the antibody occurs site-specifically between an amino acid residue of Xaa2 and a Lys252 residue according to the Eu numbering in the humanized antibody of the present invention. These Lys residues are present in the Fc region of the humanized antibody of the present invention.

The method for binding the antibody-modification peptide to the antibody can be performed, for example, by dispersing the aforementioned antibody-modification peptide, an antibody, a crosslinking agent, and a catalyst as necessary in an appropriate buffer at not less than 10°C and not more than 30°C. The reaction time may be about not less than 10 min to 2 hr or less. The molar ratio at the time of reaction of the peptide and the antibody is preferably not less than 1/5, more preferably not less than 1/3, further preferably not less than 1/1.5 as the lower limit of the antibody/peptide, and the upper limit is preferably not more than 20/1, more preferably not more than 10/1, further preferably not more than 5/1, further more preferably not more than 1/1, particularly preferably not more than 1/1.7. For example, the range of not less than 1/5 and not more than 20/1 is preferable, and not less than 1/1.5 and not more than 1/1.7 is more preferable.

The peptide-modified antibody obtained through the above steps is a mixture containing an antibody in which one molecule of antibody-modification peptide is bound to one molecule of an antibody (hereinafter to be referred to as "monovalent antibody") and an antibody in which two molecules of antibody-modification peptide are bound to one molecule of an antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is preferable to use a column packed with various fillers, and it is more preferable to use a column packed with a filler suitable for separation and purification of proteins such as antibody and the like.

The shape of the carrier of the filler suitable for separation and purification of proteins such as antibody and the like includes gel (e.g., column gel), particle, bead, nanoparticle, microparticle, macrobead, and the like. The materials of the carrier include magnetic substance, latex, agarose, glass, cellulose, sepharose, nitrocellulose, polystyrene, and other polymeric materials. Specific example is an IgG-BP column in which the aforementioned antibody-modification peptide is bound to a column gel (see WO 2021/080008).

An IgG-BP column is a column in which an IgG-binding peptide is immobilized. Divalent antibody cannot bind to the column because the binding sites are already occupied by IgG-binding peptides, and only monovalent antibodies show affinity for the column. Using the IgG-BP column and utilizing the difference in the interaction with respective antibody-modification peptides, the first antibody composition containing relatively large amounts of the unmodified antibody and the monovalent antibody and the second antibody composition containing a relatively large amount of the divalent antibody can be respectively separated and purified. In one preferred embodiment, the molar ratio of unmodified antibody and monovalent antibody in the first antibody composition is 4-47:53-96, preferably 4-30:70-96, more preferably 4-20:80-96, further preferably 4-10:90-96.

The first antibody composition or the second antibody composition separated and purified in this manner may be used as it is for the click reaction in the subsequent step (B), or may be used for the click reaction in step (B) after adjusting the protein concentration of the peptide-modified antibody contained.

The separation and purification of peptide-modified antibody used for click reaction in step (B) is explained below as an example.

A peptide-modified antibody is subjected to a click reaction in step (B) after an antibody modification step in which a modified antibody is obtained by site-specifically modifying an Fc region of an antibody by a linker provided with an antibody-modification peptide (antibody-modification linker), and an antibody purification step in which the modified antibody is purified using the aforementioned carrier with an immunoglobulin-binding protein immobilized thereon. In addition, the antibody purification step further includes a retention step of retaining the modified antibody retained on the carrier, a washing step of washing the modified antibody not retained on the carrier, and an elution step of eluting the modified antibody retained on the carrier in the retention step.

More specifically, in the antibody modification step, a modified antibody is obtained as a mixture containing an unmodified antibody not modified by an antibody-modification linker, a monovalent antibody, and a divalent antibody and, in the antibody purification step, the first antibody composition containing relatively large amounts of the unmodified antibody and the monovalent antibody and the second antibody composition containing a relatively large amount of the divalent antibody are respectively eluted utilizing the difference in the interaction of the unmodified antibody, monovalent antibody and divalent antibody with immunoglobulin-binding proteins. That is, in the retention step and washing step among the antibody purification steps, the second antibody composition containing a relatively large amount of peptide-modified antibody (divalent antibody) having a low degree of interaction with immunoglobulin-binding proteins is eluted and, in the elution step among the antibody purification steps, the first antibody composition containing a relatively large amount of peptide-modified antibody (unmodified antibody and monovalent antibody) having a high degree of interaction with immunoglobulin-binding proteins is eluted. As used herein, "containing a relatively large amount of unmodified antibody and monovalent antibody" means that the total amount of unmodified antibody and monovalent antibody contained in the first antibody composition is larger than that of the divalent antibody contained in the antibody composition, preferably that the total amount of the unmodified antibody and monovalent antibody is not less than 55%, not less than 63%, not less than 70%, not less than 80%, or not less than 90%, of the total amount (100%) of the unmodified antibody and modified antibody contained in the antibody composition. In addition, "containing a relatively large amount of divalent antibody" means that the amount of divalent antibody contained in the second antibody composition is larger than that of the monovalent antibody contained in the antibody composition, preferably that the amount of divalent antibody is not less than 55%, not less than 63%, not less than 70%, not less than 80%, or not less than 90%, of the total amount (100%) of the unmodified antibody and modified antibody contained in the antibody composition.

In the retention step, a solution containing the mixture of the unmodified antibody, monovalent antibody and divalent antibody obtained in the antibody modification step is added to a column, and unmodified antibody and monovalent antibody retained on the carrier are retained on the column and the divalent antibody not retained on the carrier is allowed to pass through. The solution that passed through in the retention step constitutes a part of the second antibody composition. To facilitate retention of the unmodified antibody and monovalent antibody on the column and to prevent aggregation or denaturation of these, it is preferable to dilute the mixed solution of the peptide-modified antibody with an appropriate dilution solvent and add same to the column. The dilution solvent is not particularly limited as long as the peptide-modified antibody dissolves and does not easily aggregate or denature in the solvent, and water, saline, buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. When a buffer is used as a dilution solvent, the concentration of the buffering agent is not less than 10 mmol/L, preferably not less than 15 mmol/L, more preferably not less than 20 mmol/L as the lower limit, and not more than 1000 mmol/L, preferably not more than 500 mmol/L, more preferably not more than 100 mmol/L as the upper limit. In addition, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the washing step, the modified antibody remaining in the column is eluted from the column by using a wash solvent. The solution that passed through the column in the aforementioned retention step and the solution eluted from the column in the washing step contain a relatively large amount of the divalent antibody, and therefore, these can be combined and used as the second antibody composition.

The wash solvent is not particularly limited as long as the peptide-modified antibody dissolves and does not easily aggregate or denature in the solvent, and it is a buffer having appropriate pH buffering capacity, and buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. The concentration of the buffering agent used as the wash solvent is not less than 20 mmol/L, preferably not less than 30 mmol/L as the lower limit, and not more than 200 mmol/L, preferably not more than 70 mmol/L as the upper limit. The pH of the wash solvent is not less than 4.0, preferably not less than 4.5, more preferably not less than 4.8 as the lower limit, and not more than 7.4, preferably not more than 6.0, more preferably not more than 5.2 as the upper limit. Furthermore, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the elution step, the modified antibody retained on the carrier is eluted from the column by using an elution solvent. That is, the first antibody composition containing a relatively large amount of unmodified antibody and monovalent antibody is eluted from the column by using an elution solvent.

As the elution solvent, a buffer such as sodium acetate buffer, ammonium acetate buffer, citrate buffer and the like, and the like can be used. In addition, to reduce non-specific binding to the antibody-modification linker, unmodified antibody and modified antibody column carrier, the elution solvent may contain an additive such as sodium chloride, potassium chloride and the like. The concentration of the additive contained in the elution solvent is not particularly limited and may be, for example, 0.15 mol/L.

When the elution solvent contains a buffering agent, the concentration of the buffering agent is not less than 20 mmol/L, preferably not less than 30 mmol/L as the lower limit, and not more than 200 mmol/L, preferably not more than 70 mmol/L as the upper limit. In addition, to weaken the interaction between the unmodified antibody and monovalent antibody, and the immunoglobulin-binding protein, and to prevent denaturation and aggregation of the antibody, the pH of the elution solvent is not less than pH 3.0 as the lower limit, and not more than pH 4.2 as the upper limit.

The first antibody composition or the second antibody composition obtained in the antibody purification step may be used as it is for the click reaction in the subsequent step (B), or may be used for the click reaction in step (B) after adjusting the protein concentration of the peptide-modified antibody contained.

The click reaction in step (B) is performed between the' first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the order of addition of these does not matter. For example, one of the complex and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferred, and the range of not less than 0.1 mL and not more than 10 mL is more preferred.

As the concentrations of the chelating agent and the antibody in the reaction mixture, each independently, the lower limit at the start of step (B) is preferably not less than 0.001 µmol/L, more preferably not less than 0.01 µmol/L, further preferably not less than 0.1 µmol/L, further more preferably not less than 1.0 µmol/L, and the upper limit is preferably not more than 1000 µmol/L, more preferably not more than 100 µmol/L. For example, the range of not less than 0.1 µmol/L and not more than 1000 µmol/L is preferred, and the range of not less than 1 µmol/L and not more than 100 µmol/L is more preferred, from the aspect of the yield of the desired chelate conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferred, and the range of not less than 10 min and not more than 20 hr is more preferred.

In the radiolabeled antibody produced by steps (A) and (B), a specific site of an antibody that specifically binds to an antigen (e.g., the lysine residue in the Fc region of antibody) is specifically modified with a chelating agent. This radiolabeled antibody comprises one or two molecules of the aforementioned chelating agent per one molecule of the antibody. The chelating agent site-specifically modifies the Fc region of the antibody via a linker. The linker is constituted of a chelate linker that connects to a chelating agent, a first atomic group that connects to the linker, a second atomic group that can perform click reaction with the first atomic group, and an antibody-modification linker that connects to the second atomic group (including an antibody-modification peptide represented by the above-mentioned formula (i)). Therefore, the linker has a chemical structure derived from the first atomic group and the second atomic group. As such chemical structure, a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

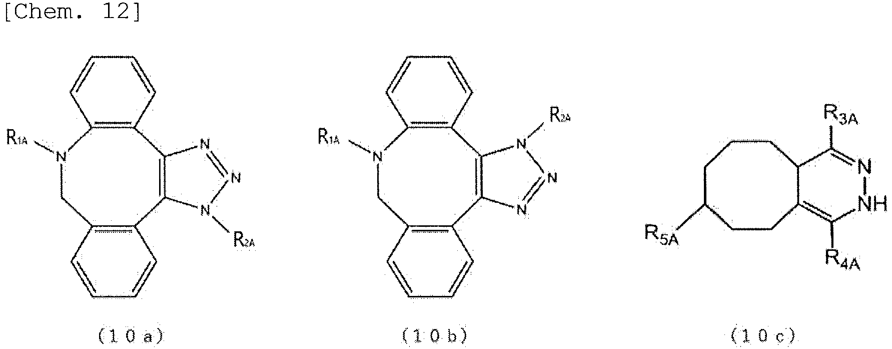

In the formula (10a) and the formula (10b), R_{1A} is a binding site with a chelate linker, and R_{2A} is a binding site with an antibody-modification linker. In the formula (10c), one of R_{3A} and R_{4A} is a hydrogen atom, a methyl group, a phenyl group or a pyridyl group, and the other is a binding site with a chelate linker, and R_{5A} is a binding site with an antibody-modification linker.

Accordingly, a solution of a crude product of a radiolabeled antibody is obtained.

### (Step 2) Step of contacting the solution of the crude product obtained in Step 1 with a cation exchange material to adsorb the radiolabeled antibody onto the cation exchange material

### (2-1) cation exchange material

The "cation exchange material" refers to a negatively charged solid phase having free cations that are exchanged for cations in a solution passing over or through the solid phase. The charge can be obtained by attaching one or more charged ligands to the solid phase, for example, by covalent bonding. The charge can be an inherent property of the solid phase (e.g., silica). In some embodiments, the cation exchange material may be a membrane, a monolith, or a resin. In the present invention, a preferred cation exchange material is one that has a charged group on the resin support. The charged group is a carboxyl group, a sulfo group, or a phosphate group, but may also include, for example, a sulfonate group, a carboxymethyl group, a sulfoisobutyl group, a sulfoethyl group, a carboxyl group, a sulfopropyl group, a sulfonyl group, a sulfoxyethyl group, a benzenesulfonic acid group, or an orthophosphate group, and is more preferably a sulfopropyl group or a carboxymethyl group. The resin carrier may, for example, be carboxymethylcellulose or agarose. In some of the above-mentioned embodiments, the cation exchange material is a cation exchange chromatography material, and may be provided in the form of a cation exchange chromatography column in which the material is packed in a column. Commercially available cation exchange materials include carboxymethylcellulose, BAKERBOND ABX^{™}, sulfopropyl (SP) immobilized on agarose (e.g., SP-SEPHAROSE FAST FLOW^{™}, SP-SEPHAROSE FAST FLOW XL^{™}, or SP-SEPHAROSE HIGH PERFORMANCE^{™} from GE Healthcare, HiTrap SP FF^{™} from Cytiva, etc.), CAPTO S^{™} (GE Healthcare), FRACTOGEL-SO3^{™}, FRACTOGEL-SE HICAP^{™}, and FRACTOPREP^{™} (EMD Merck), sulfonyl immobilized on agarose (e.g., S-SEPHAROSE FAST FLOW^{™} from GE Healthcare), and SUPER SP^{™} (Tosoh Biosciences).

The cation exchange material can be washed, and is preferably washed (so-called equilibration) before the below-mentioned "contact between the radiolabeled antibody and the cation exchange material". The equilibration liquid is a liquid used to equilibrate the cation exchange material before contacting a solution of a crude product of a radiolabeled antibody with the cation exchange material. As the equilibration liquid, it is preferable to use a solution having the same composition as the "eluent" used in Step 3, and it is more preferable to use two types of solution: a solution having the same composition as the washing solution described below in "(2-3) Washing step", and a solution having the same composition as the "eluent" used in Step 3. The pH of the equilibration liquid is in the range of 4 or more and 8 or less, and preferably 5 or more and 6 or less.

The amount of the above-mentioned cation exchange material to be used is preferably 0.01 mL or more and preferably 1 mL or less, per 1 mg of antibody.

### (2-2) Contact between the radiolabeled antibody and the cation exchange material

In Step 2, a solution of a crude product of a radiolabeled antibody obtained in Step 1 is contacted with a cation exchange material (mentioned above) to adsorb the radiolabeled antibody onto the cation exchange material. As used herein, "contact" or "contacted" refers to exposing the radiolabeled antibody to a cation exchange material under appropriate conditions such that the radiolabeled antibody is reversibly immobilized (i.e., adsorbed) in or onto the cation exchange material due to ionic interactions between the radiolabeled antibody and the charged group or charged groups of the cation exchange material. For example, in the embodiment of a cation exchange chromatography column in which the cation exchange material is packed in a column, the contact refers to passing a solution of a crude product of a radiolabeled antibody through the cation exchange chromatography column.

The solution of a crude product of a radiolabeled antibody is not particularly limited as long as it is prepared such that the radiolabeled antibody can be adsorbed onto the cation exchange material. For example, the pH of the solution of the crude product of the radiolabeled antibody can be set lower than the isoelectric point of the radiolabeled antibody. In addition, the below-mentioned "positively charged additive" (arginine as one example) may be added to the solution of the crude product of the radiolabeled antibody. In this case, the concentration of the positively charged additive (arginine as one example) contained in the solution of the crude product of the radiolabeled antibody can be preferably set to 30 mmol/L or less.

As a result, a radiolabeled antibody is adsorbed onto a cation exchange material.

In one embodiment of the present invention, it is preferable to wash the cation exchange material, to which the radiolabeled antibody is adsorbed, with a washing solution (hereinafter also to be referred to as the "washing step") after performing Step 2 and before performing Step 3 described below.

### (2-3) Washing step

The washing solution used in this step is used to remove one or more impurities from the cation exchange material without substantially eluting the desired radiolabeled antibody. The impurities are substances other than the desired radiolabeled antibody, and include, for example, unreacted radionuclides, antibodies, and chelating agents present in the solution of the crude product of the radiolabeled antibody.

The washing solution preferably contains one or more pH adjusters, and the pH adjuster is at least one buffer selected from the group consisting of, but not limited to, histidine, 2-morpholinoethanesulfonic acid (MES), 2-[bis(2-hydroxyethyl)amino]-2-(hydroxymethyl)propane-1,3-diol (Bis-Tris), N-(2-acetamido)iminodiacetic acid (ADA), piperazine-1,4-bis(2-ethanesulfonic acid) (PIPES), N-(2-acetamido)-2-aminoethanesulfonic acid (ACES), 2-hydroxy-3-morpholinopropanesulfonic acid (MOPSO), N,N-bis(2-hydroxyethyl)-2-aminoethanesulfonic acid (BES), 3-morpholinopropane-1-sulfonic acid (MOPS), N-tris (hydroxymethyl)methyl-2-aminoethanesulfonic acid (TES), and 2-[4-(2-hydroxyethyl)-1-piperazinyl]ethanesulfonic acid (HEPES). It generally has a higher pH than the pH of the solution used when the radiolabeled antibody is contacted with the cation exchange material in the below-mentioned Step 3. The pH of the washing solution is preferably 4 or more, more preferably 5 or more, and preferably 7 or less, and more preferably 6.5 or less.

The washing step may be performed once, or can be performed 2 to 5 times when desired. Preferably, a liquid volume of at least 3 times the volume of the cation exchange material is used per one washing step.

### (Step 3) Step of eluting the radiolabeled antibody adsorbed to the cation exchange material in Step 2 with an eluent containing one or more additives

### (3-1) Eluent

In the present invention, the "eluent" refers to a solution used to elute a radiolabeled antibody from a cation exchange material onto which the antibody is adsorbed. As used herein, the eluent is designed such that the radiolabeled antibody is eluted from the cation exchange material. An additive (hereinafter also to be referred to as "first additive", corresponding to additive (A)) contained in the composition of the radioactive pharmaceutical composition, which is the final product of the production method of the present invention, is added to the eluent. The first additive is not limited as long as it is a pharmaceutical additive and elutes the radiolabeled antibody from the cation exchange material. From the aspect of increasing the radiochemical yield, it preferably contains a positively-charged additive (corresponding to additive (a1)) and a pH adjuster.

The "positively-charged additive" refers to a substance consisting of or containing a compound that is positively charged when in contact with water. The compound that becomes positively charged when in contact with water generally has a cationic group in the molecule. Examples of the cationic group include amino groups such as primary amino group, secondary amino group, and tertiary amino group, onium bases such as quaternary ammonium group and phosphonium group, amino acid residues such as arginyl group, lysyl group, histidyl group, and guanidyl group, and heterocyclic groups such as imidazole group, and preferred is a compound having a guanidine skeleton. Examples of the compound having a guanidine skeleton include arginine and arginine derivatives such as acylated arginine and agmatine, and arginine is preferred. In this case, the concentration of arginine in the eluent is preferably 10 mmol/L or more, more preferably 30 mmol/L or more, further preferably 50 mmol/L or more, and is preferably 500 mmol/L or less, more preferably 400 mmol/L or less, further preferably 300 mmol/L or less.

The positively-charged additive may contain a salt intended to increase conductivity in addition to the above-mentioned compound that becomes positively charged when in contact with water, such as arginine. Usable salts include sodium chloride, sodium acetate, potassium chloride, and the like, and at least one of these can be contained in the positively-charged additive. In the case of sodium chloride, it is preferably used at a concentration of 100 mmol/L or more, more preferably 150 mmol/L or more, preferably 300 mmol/L or less, more preferably 200 mmol/L or less.

As the "pH adjuster", a pharmaceutical additive that achieves the purpose of adjusting the eluent to a desired pH can be used, and specifically, a histidine buffer is preferred, and in addition to or instead of the histidine buffer, at least one buffer selected from the group consisting of 2-[4-(2-hydroxyethyl)-1-piperazinyl]ethanesulfonic acid (HEPES) buffer, phosphate buffer, and citrate buffer can be used.

When a histidine buffer is used as the pH adjuster, the concentration of histidine in the eluent is preferably 10 mmol/L or more, more preferably 20 mmol/L or more, further preferably 30 mmol/L or more, and preferably 300 mmol/L or less, more preferably 250 mmol/L or less, further preferably 200 mmol/L or less.

The pH of the eluent is preferably higher than 6, preferably 8 or less, and more preferably 7 or less.

The eluent of the present invention can be a composition in the formulation for a radioactive pharmaceutical composition.

### (3-2) Elution

The elution of the radiolabeled antibody adsorbed onto the cation exchange material from the material can be performed, for example, when the cation exchange material is a cation chromatography column, by passing the above-mentioned eluent through the column onto which the radiolabeled antibody is adsorbed. The passage of the eluent may be performed once, or may be performed two or three times when desired. The amount of the liquid used for one elution step is not less than three times the volume of the cation exchange material.

In the present invention, in producing a radioactive pharmaceutical composition, a diluent may be added to the eluate containing the radiolabeled antibody after performing Step 3. Since the eluent of the present invention can be a composition in the formulation, it is possible to formulate the composition without solvent exchange but by adding a diluent to the eluate. As the diluent, water for injection or physiological saline is used.

In addition, a further additive (hereinafter also to be referred to as "second additive", corresponding to additive (B)) may be added to the eluate. The second additive is not limited as long as it is a pharmaceutical additive, and examples thereof include stabilizer, surfactant, antioxidant, chelating agent, and pH adjuster. The second additive can be added, for example, by dissolving same in a diluent.

### (3-3) Liquid pharmaceutical composition

By performing the above-mentioned Step 1 to Step 3, and optionally a washing step between Step 2 and Step 3, and a dilution step after Step 3 when desired, a "liquid radioactive pharmaceutical composition containing a radiolabeled antibody, in which the antibody is labeled with the radionuclide, as an active ingredient and one or more additives" is obtained. As used herein, "liquid" refers to being a liquid or fluid state under general conditions (e.g., in the atmosphere, at room temperature/ordinary temperature), and also includes a state having some viscosity. Specific examples include, but are not limited to, solution, suspension, dispersion, emulsion, and the like. A liquid pharmaceutical composition can be produced, for example, by dissolving a chelate conjugate of the present invention, which is produced by the aforementioned method, in a solvent mainly containing water and almost isotonic with the living body. In this case, the composition may contain other pharmaceutically acceptable components as necessary.

An effective amount of the liquid pharmaceutical composition is orally or parenterally, for example, intravenously, subcutaneously, intraperitoneally, intramuscularly, or the like, administered to a living body, and is used for treatment of a disease, diagnosis of a disease, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

By selecting a radionuclide having a therapeutic effect, specifically α particle emitting nuclide, the liquid pharmaceutical composition of the present invention can be used for internal radionuclide therapy. In internal radionuclide therapy, a radiopharmaceutical is administered intravenously or orally, this radiopharmaceutical is accumulated in a lesion site such as a primary cancer lesion or a metastatic lesion, and the cancer cells in the lesion site are destroyed by radiation emitted from the radiopharmaceutical. Therefore, the liquid pharmaceutical composition of the present invention can be preferably used for internal radionuclide therapy of cancer. In this case, the administration amount and dosage of the pharmaceutical composition are appropriately selected according to the kind of the radionuclide, the effectiveness of the active ingredient, the form and route of administration, the stage of progression of the disease (particularly cancer), body shape, body weight, age of the patient, and the kind and amount of other therapeutic agent to be used in combination for the disease.

Furthermore, as the administration amount and dosage of the pharmaceutical composition, a human equivalent dose (HED), which is calculated by converting the dose for a mouse to a human based on the body surface area, can be used. HED is determined by the following formula. HED=animal dose in MBq/kg × (animal weight in kg/human weight in kg)0.33

For example, when the HED for a human weighing 60 kg is calculated using the dose for a mouse weighing 20 g and when the radionuclide is iodine-131, it can generally be administered at not more than 100 MBq/kg per dose. The effect can be exhibited even at a dose of not more than 50 MBq/kg one time. For example, when the radionuclide is yttrium-90, it can generally be administered at not more than 50 MBq/kg per dose. The effect can be exhibited even at a dose of not more than 30 MBq/kg one time. For example, when radionuclide is lutetium-177, it can generally be administered at not more than 50 MBq/kg per dose. The effect can be exhibited even at a dose of not more than 40 MBq/kg one time. Such dosage is an example of one embodiment of the present invention, and those skilled in the art can make appropriate conversion according to the above-mentioned formula, based on the body weights of mouse and human.

When radionuclide is iodine-131, the toxicity to the thyroid gland, where iodine accumulates physiologically, can be reduced by pre-administration of a non-radioactive iodine preparation (stable iodine preparation). As a stable iodine preparation, potassium iodide, potassium iodate, and the like can be used.

In addition, as another embodiment of the present invention, when a radionuclide that emits positron or gamma ray from a β-ray emitting nuclide is used as the radionuclide in the aforementioned radiolabeled antibody, the obtained radioactive pharmaceutical composition may be used for cancer diagnosis in internal radionuclide therapy for cancer. The diagnostic pharmaceutical composition for cancer of the present invention may be used for diagnosis before performing RI internal therapy for cancer, or may be used for diagnosis after performing an internal radionuclide therapy for cancer. Using the radiopharmaceutical for diagnosis before conducting an internal radionuclide therapy for cancer, selection of treatment can be performed as to whether or not to perform an internal radionuclide therapy for cancer using the liquid pharmaceutical composition of the present invention provided with a radionuclide that emits β-rays. Using the composition for diagnosis after conducting an internal radionuclide therapy for cancer, moreover, whether or not internal radionuclide therapy for cancer using the liquid pharmaceutical composition of the present invention provided with a radionuclide that emits β-rays is effective can be judged, and a treatment plan including an increase or a decrease of dose and the like can be optimized.

### (2) Purification method of radiolabeled antibody

The present invention provides a method for purifying a radiolabeled antibody, comprising:
(Step A) a step of labeling an antibody with a radionuclide to obtain a solution of a crude product of a radiolabeled antibody;
(Step B) a step of contacting the solution of the crude product obtained in the aforementioned Step A with a cation exchange material to adsorb the aforementioned radiolabeled antibody onto the aforementioned cation exchange material; and
(Step C) a step of eluting the aforementioned radiolabeled antibody adsorbed onto the cation exchange material in the aforementioned Step B with an eluent containing one or more additives.

Step A can be performed in the same manner as in the above-mentioned Step 1 of "(1) Pharmaceutical composition and a production method thereof".

Step B can be performed in the same manner as in the above-mentioned Step 2 of "(1) Pharmaceutical composition and a production method thereof".

Step C can be performed in the same manner as in the above-mentioned Step 3 of "(1) Pharmaceutical composition and a production method thereof".

The eluate obtained through step C contains the radiolabeled antibody at a high concentration, and therefore a high radiochemical yield is expected. In addition, high radiochemical purity can be maintained.

The liquid pharmaceutical composition of the present invention when stored at room temperature has a radiochemical purity of a certain level or above at the time of expiry of a period that is a multiple of not less than 1 and not more than 5 of the half-life, based on the half-life of the radioactive metal nuclide constituting the radiolabeled antibody contained in the liquid pharmaceutical composition. When the above-mentioned radioactive metal nuclide is a positron-emitting nuclide (e.g., Zr-89), the radiochemical purity of the conjugate is preferably not less than 90%, more preferably not less than 95%, when stored at room temperature for 7 days after the completion of the production. When the radioactive metal nuclide is an α particle nuclide (e.g., Ac-225), the radiochemical purity of the conjugate is preferably not less than 90%, more preferably not less than 95%, when stored at room temperature for 14 days from the completion of the production. The "room temperature" in the present specification preferably refers to the "ordinary temperature" defined in the Japanese Pharmacopoeia, which is specifically 15 to 25°C. In addition, during storage or transportation to a medical site, the temperature may deviate from the temperature set in the storage conditions, and therefore, it is preferable for the liquid pharmaceutical composition to have little change in the radiochemical purity even when temperature deviation occurs.

As used herein, the radiochemical purity refers to the percentage of the peak radioactivity (count) corresponding to the conjugate with respect to the total radioactivity (count) detected when the sample is analyzed with a commercially available radiation detector. High performance liquid chromatography and thin-layer chromatography can be used for the analysis of radiochemical purity, and thin-layer chromatography is preferably used. More preferably, thin-layer chromatography is used under the conditions described in the below-mentioned Examples.

The above-mentioned embodiment of the present invention includes the following technical ideas.
[1] A method for producing a liquid radioactive pharmaceutical composition containing, as an active ingredient, a radiolabeled antibody obtained by labeling an antibody with a radionuclide, and containing one or more additives (A), comprising:
   (Step 1) a step of labeling an antibody with a radionuclide to obtain a solution of a crude product of the aforementioned radiolabeled antibody;
   (Step 2) a step of contacting the solution of the crude product obtained in the aforementioned Step 1 with a cation exchange material to adsorb the aforementioned radiolabeled antibody onto the aforementioned cation exchange material; and
   (Step 3) a step of eluting the aforementioned radiolabeled antibody adsorbed onto the aforementioned cation exchange material in the aforementioned Step 2 with an eluent containing the aforementioned additive (A).
[2] The method of [1], wherein the aforementioned radioactive pharmaceutical composition contains a second additive (B) in addition to the first additive which is the aforementioned additive (A), and a step of adding a diluent containing the aforementioned second additive (B) to the eluate containing the aforementioned radiolabeled antibody is performed after practicing Step 3.
[3] The method of [1] or [2], wherein the radionuclide is a radioactive metal nuclide or a radioactive halogen nuclide.
[4] The method of [1] or [2], wherein the radionuclide is Al¹⁸F, ⁸⁹Zr, ⁹⁰Y, ¹¹¹In , ¹⁷⁷Lu, or ²²⁵Ac.
[5] The method of any of [1] to [4], wherein the aforementioned cation exchange material has a sulfopropyl group or a carboxymethyl group as an electric charge group.
[6] The method of any of [1] to [5], wherein the aforementioned cation exchange material is packed in a column.
[7] The method of any of [1] to [6], wherein a step of washing the cation exchange material by passing a washing solution through the aforementioned cation exchange material onto which the aforementioned radiolabeled antibody is adsorbed is performed before practicing the aforementioned Step 3.
[8] The method of [7], wherein the aforementioned washing solution contains one or more pH adjusters.
[9] The method of [8], wherein the aforementioned pH adjuster is at least one buffer selected from the group consisting of histidine, 2-morpholinoethanesulfonic acid (MES), 2-[bis(2-hydroxyethyl)amino]-2-(hydroxymethyl)propane-1,3-diol (Bis-Tris), N-(2-acetamido)iminodiacetic acid (ADA), piperazine-1,4-bis(2-ethanesulfonic acid) (PIPES), N-(2-acetamido)-2-aminoethanesulfonic acid (ACES), 2-hydroxy-3-morpholinopropanesulfonic acid (MOPSO), N,N-bis(2-hydroxyethyl)-2-aminoethanesulfonic acid (BES), 3-morpholinopropane-1-sulfonic acid (MOPS), N-tris (hydroxymethyl)methyl-2-aminoethanesulfonic acid (TES), and 2-[4-(2-hydroxyethyl)-1-piperazinyl]ethanesulfonic acid (HEPES).
[10] The method of any of [7] to [9], wherein the aforementioned washing solution has a pH of 4 to 7.
[11] The method of any of [1] to [10], wherein the aforementioned radioactive pharmaceutical composition comprises a positively-charged additive (a1) and a pH adjuster as the aforementioned additive (A), and
   the aforementioned eluent comprises the aforementioned positively-charged additive (a1) and the aforementioned pH adjuster contained in the aforementioned radioactive pharmaceutical composition.
[12] The method of [11], wherein the aforementioned positively-charged additive (a1) comprises a compound having a guanidine skeleton.
[13] The method of [12], wherein the aforementioned compound having a guanidine skeleton is at least one compound selected from the group consisting of arginine, acylated arginine, and agmatine.
[14] The method of [12] or [13], wherein the aforementioned positively-charged additive (a1) further comprises at least one compound selected from the group consisting of sodium chloride, potassium chloride, and sodium acetate.
[15] The method of any of [11] to [14], wherein the aforementioned pH adjuster comprises at least one buffering agent selected from the group consisting of histidine, 2-[4-(2-hydroxyethyl)-1-piperazinyl]ethanesulfonic acid (HEPES), phosphoric acid, and citric acid.
[16] The method of any of [1] to [15], wherein the aforementioned eluent containing the aforementioned additive (A) comprises a histidine buffer containing arginine.
[17] The method of [16], wherein the aforementioned eluent comprises 10 - 500 mmol/L of arginine and 10 - 300 mmol/L of histidine.
[18] The method of any of [1] to [17], wherein the aforementioned eluent has 6<pH≤8.
[19] A method for purifying a radiolabeled antibody, comprising:
   (Step A) a step of labeling an antibody with a radionuclide to obtain a solution of a crude product of a radiolabeled antibody;
   (Step B) a step of contacting the solution of the crude product obtained in the aforementioned Step A with a cation exchange material to adsorb the aforementioned radiolabeled antibody onto the aforementioned cation exchange material; and
   (Step C) a step of eluting the aforementioned radiolabeled antibody adsorbed onto the cation exchange material in the aforementioned Step B with an eluent containing one or more additives.
[20] The method of any of [1] to [19], wherein the aforementioned radiolabeled antibody is a conjugate of a chelating agent chelated with a radionuclide and an antibody, wherein the chelating agent site-specifically modifies Fc region of the aforementioned antibody via a linker.
[21] The conjugate of [20], wherein the linker comprises an antibody-modification peptide represented by the following formula (i) which consists of not less than 13 and not more than 17 amino acid residues:

   (Xa)-Xaa₁-(Xb)-Xaa₂-(Xc)-Xaa₃-(Xd) (i)

   wherein Xa, Xb, Xc and Xd are continuous X in the number of a, continuous X in the number of b, continuous X in the number of c, and continuous X in the number of d, respectively,
   X is an amino acid residue having neither a thiol group nor a haloacetyl group in the side chain,
   a, b, c and d are each independently an integer of not less than one and not more than 5, and satisfy a+b+c+d≤14,
   Xaa₁ and Xaa₃ are each independently an amino acid residue derived from an amino acid having a thiol group in the side chain, and they are linked via a disulfide bond or their sulfide groups are linked via a linker, or
   one is an amino acid residue derived from an amino acid having a thiol group in the side chain and the other is an amino acid residue derived from an amino acid having a haloacetyl group in the side chain, and they are linked via a thioether bond, and Xaa₂ is a lysine residue, an arginine residue, a cysteine residue, an aspartic acid residue, a glutamic acid residue, 2-aminosuberic acid, or diamino propionic acid.
[22] The method of [20] or [21], wherein the linker has a structure represented by the above-mentioned formula (10a) or the above-mentioned formula (10b).
[23] The method of any of [1] to [22], wherein the aforementioned antibody is a human antibody.
[24] The method of any of [1] to [22], wherein, in the aforementioned radiolabeled antibody, one molecule or two molecules of an antibody-modification peptide is/are bonded to one molecule of the antibody.

The present invention is described in detail in the following with reference to Example and the like, but the present invention is not limited to these.

### [Example]

### Production Example 1: Site-specific antibody modification by peptide linker

### (1) Antibody modification step

An antibody-modification peptide was produced by the method described in WO 2017/217347 to obtain a peptide containing 17 amino acid residues represented by the following formula (P3). The amino acid sequence of this peptide was the same as the sequence in SEQ ID NO: 10 in which Xaa₂ is a lysine residue, and the side chain terminal amino group of the lysine residue was modified with the structure represented by R1. In addition, two cysteine residues formed a disulfide bond with each other, and the N-terminus of the peptide was bound to ethyl azide as an atomic group containing an azide group as the second atomic group via a linker structure having diglycolic acid and eight PEGs. 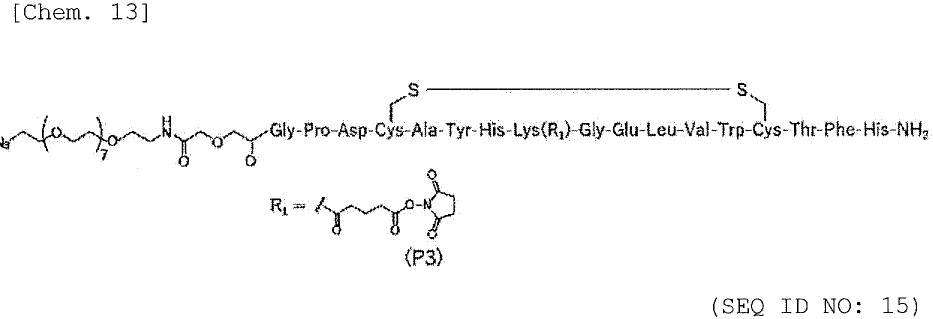 (in the formula (P3), Gly is glycine, Pro is proline, Asp is aspartic acid, Cys is cysteine, Ala is alanine, Tyr is tyrosine, His is histidine, Glu is glutamic acid, Leu is leucine, Val is valine, Trp is tryptophan, and Phe is phenylalanine.)

This peptide was mixed with the antibody drug shown in Table 2 in sodium acetate buffer (pH 6.0) and the mixture was reacted at room temperature for 30 min to obtain a solution containing peptide-modified antibody. This peptide-modified antibody is an antibody in which the Fc region of each antibody has been site-specifically modified with the above-mentioned peptide.

**[Table 2]**

| name of antibody used | trade name | manufacturer |
|---|---|---|
| trastuzumab | Herceptin | Roche |
| panitumumab | Vectibix | Amgen |
| rituximab | MabThera | Roche |
| bevacizumab | avastin | Roche |

### (2) Peptide-modified antibody separation step

This peptide-modified antibody was diluted with 20 mmol/L sodium acetate buffer (pH 6.0) and then passed through an IgG-BP column prepared according to the method described in Example 2 of WO 2020/075670 to obtain an antibody composition containing relatively large amounts of unlabeled antibody and monovalent antibody. The solution containing relatively large amounts of unlabeled antibody and monovalent antibody was subjected to the labeling step described below. The purity of the recovered peptide-modified antibody was analyzed by hydrophobic column chromatography, and a peptide-modified antibody was obtained with the recovery amount and unmodified antibody:monovalent antibody:divalent antibody ratio shown in Table 3.

**[Table 3]**

| name of antibody used | monovalent antibody yield (mg) | Unmodified antibody:monovalent antibody:divalent antibody ratio |
|---|---|---|
| trastuzumab | 25.02 | 4.18:95.82:0 |
| panitumumab | 21.03 | 13.9:86.1:0 |
| rituximab | 13.90 | 17.82:82.18:0 |
| bevacizumab | 18.24 | 9.85:90.15:0 |

The hydrophobic column chromatography conditions are as follows.
column: BioPro HIC BF (4.6 mm×10cm, 4.0 µm)
detector: ultraviolet absorption spectrophotometer

(measurement wavelength: 280 nm)
mobile phase A: 50 mmol/L phosphate buffer (pH 7.0) containing 1.5 mol/L ammonium sulfate
mobile phase B: 50 mmol/L phosphate buffer (pH 7.0)
mobile phase C: water
feeding of mobile phase: The mixing ratio of mobile phase A, mobile phase B, and mobile phase C is changed as follows to control the concentration gradient.

**[Table 4]**

| time after injection (min) | mobile phase A (% by volume) | mobile phase B (% by volume) | mobile phase C (% by volume) |
|---|---|---|---|
| 0 - 5.0 | 100 | 0 | 0 |
| 5.0 - 30.0 | 100→0 | 0→100 | 0 |
| 30.0 - 30.5 | 0 | 100→0 | 0→100 |
| 30.5 - 40.0 | 0 | 0 | 100 |
| 40.0 - 40.5 | 0→100 | 0 | 100→0 |
| 40.5 - 50.0 | 100 | 0 | 0 |

| | | | |
|---|---|---|---|
| flow rate: 1.0 mL per minute | | | |

### Example 1: Production of radioactive pharmaceutical composition with ⁸⁹Zr-labeled rituximab as active ingredient

### (1) Complex formation step

The structure of the chelating moiety (DOTA-GA-DBCO) used in this Example is shown in the following formula (L1-5). A ⁸⁹Y target was irradiated with protons and dissolved in a 0.1 mol/L aqueous hydrochloric acid solution to prepare a ⁸⁹Zr ion-containing solution (radioactivity concentration: 4200 MBq/mL, liquid volume: 0.100 mL). The solvent was distilled off under heating conditions. The chelating moiety was dispersed in 100 mmol/L acetic acid-sodium acetate buffer (pH 5.0) to obtain a dispersion containing 0.3 mmol/L of the chelating moiety (⁸⁹Zr chelating moiety dispersion). 0.074 mL of this ⁸⁹Zr dispersion and 0.099 mL of a 150 mmol/L gentisic acid solution (dissolved in 100 mmol/L acetic acid-sodium acetate buffer (pH 5.0)) were added to the radioactive metal source from which the solvent had been distilled off, and they were reacted under heating conditions to obtain a ⁸⁹Zr complex solution. The molar ratio of the chelating moiety to the radioactive metal ion was chelating moiety:⁸⁹Zr ion = about 78.1:1, the heating temperature of the reaction mixture was 70°C, and the heating time was 60 min.

The radiochemical purity of the ⁸⁹Zr complex in the ⁸⁹Zr complex solution was measured by TLC analysis, and a ⁸⁹Zr complex was obtained with a radiochemical purity of 96.5%. The TLC analysis was performed by thin layer chromatography (manufactured by Agilent, model number: SGI0001, developing solvent: acetonitrile:water (volume ratio 1:1)) using a radio γ-TZC analyzer (manufactured by Raytest, MODEL GITA Star PS), and the percentage of the radioactivity (counts) of the peak detected near the solvent tip relative to the total radioactivity (counts) detected was taken as the radiochemical purity (%).

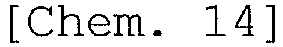

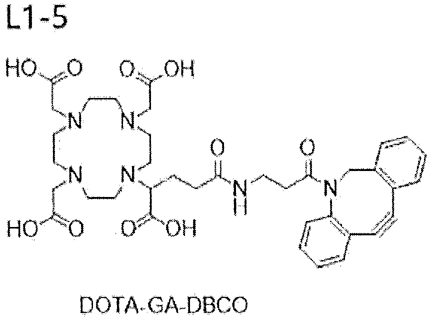

### (2) Labeling step

The solution of the ⁸⁹Zr complex obtained through the aforementioned step (1) was mixed with a solution containing the peptide-modified rituximab (monovalent antibody) produced through Production Example 1, and a click reaction was performed at 37°C for 2.0 hr to obtain ⁸⁹Zr-labeled rituximab. The radiochemical purity was measured in the same manner as in step (1) except that an acetonitrile:0.1 mol/L EDTA solution (volume ratio 1:1) was used as the developing solvent, and the percentage of the radioactivity (counts) of the peak detected near the point of origin relative to the total radioactivity (counts) detected was used as the radiochemical purity (%), and it was found that ⁸⁹Zr-labeled rituximab was obtained with a radiochemical purity of 65.8%.

### (3) Purification step

A solution (120 µL) containing ⁸⁹Zr-labeled rituximab obtained in step (2), the ⁸⁹Zr complex solution (80 µL) obtained in the aforementioned step (1), and a ⁸⁹Zr hydrochloric acid solution (5 µL) were blended to produce a pseudoreaction solution for purification study, containing 26.5% of ⁸⁹Zr-labeled rituximab and 10.1% of a free ⁸⁹Zr component (the percentage of total radioactive components in the pseudoreaction solution detected by TLC is taken as 100%).

A column (HiTrap^{™} SP FF 1 mL) packed with a cation exchange resin having a sulfopropyl group as a charged group on an agarose carrier was equilibrated with 5 mL of 20 mmol/L histidine buffer (pH 5.5) (hereinafter referred to as "His5.5"), 5 mL of 100 mmol/L arginine-containing 50 mmol/L histidine buffer (pH 6.1) (hereinafter referred to as "RH6.1"), and 10 mL of His5.5, in this order

10 µL (11.7 MBq) of a pseudoreaction solution was passed through the above-mentioned equilibrated column, and the ⁸⁹Zr-labeled antibody was retained in the column. Thereafter, 5 mL of His5.5 was passed through the column twice for washing. Then, 3 mL of RH6.1 was passed through, and the eluate (1.16 MBq) was collected. The radiochemical purity of the eluate was analyzed by TLC in the same manner as in step (2), and the radioactivity of each was measured and the radiochemical yield of the target ⁸⁹Zr-labeled rituximab was calculated. As a result, the radiochemical yield was 37.3% and the radiochemical purity was 78.4%. The radiochemical yield is a value expressed as a percentage, which is obtained by dividing the radioactivity of the ⁸⁹Zr-labeled antibody contained in 3 mL of the eluate by the radioactivity of the ⁸⁹Zr-labeled antibody in the pseudoreaction solution passed through the column.

### Comparative Example 1: Production of radioactive pharmaceutical composition with ⁸⁹Zr-labeled rituximab as active ingredient

A cation exchange resin was equilibrated with 5 mL of His5.5, 5 mL of 160 mmol/L NaCl-containing 19 mmol/L MES buffer (pH 5.5) (hereinafter to be referred to as "NaMES5.5") in this order, and in the same manner as in Example 1 except that 3 mL of NaMES5.5 was used as an eluent, ⁸⁹Zr-labeled rituximab was purified from a pseudoreaction solution. The MES buffer has never been used as an additive in pharmaceutical products. As a result, the radiochemical yield was 13.6% and the radiochemical purity was 50.9%.

### Examples 2 - 5: Production of radioactive pharmaceutical compositions with various radiolabeled antibodies as active ingredients

### (1) Complex formation step

Performed in the same manner as in Example 1, step (1).

### (2) Labeling step

The solution of the ⁸⁹Zr complex obtained through the aforementioned step (1) was mixed with a solution containing each peptide-modified antibody (monovalent antibody) produced through Production Example 1, and a click reaction was performed at 37°C for 2.0 hr to obtain ⁸⁹Zr-labeled antibody.

### (3) Purification step

A column (HiTrap SP FF 1 mL) packed with a cation exchange resin having a sulfopropyl group as a charged group on an agarose carrier was equilibrated with 5 mL of His5.5, 5 mL of RH6.1, and 10 mL of His5.5. The total amount of the reaction mixture obtained in step (2) was passed through the above-mentioned equilibrated column, and the ⁸⁹Zr-labeled antibody was retained in the column. Then, 1 mL of His5.5 was passed through the above-mentioned column 5 times for washing. 1 mL of RH6.1 was passed through this column 3 times, and the eluate was collected. The radiochemical purity of the eluate was analyzed by TLC in the same manner as in step (2), and the radioactivity of each was measured and the radiochemical yield of the target ⁸⁹Zr-labeled antibody was calculated. The radiochemical yield is a value expressed as a percentage, which is obtained by dividing the radioactivity of the ⁸⁹Zr-labeled antibody contained in 3 mL of the eluate by the radioactivity of the ⁸⁹Zr-labeled antibody in the reaction solution passed through the column. The radiochemical yield radiochemical purity of the obtained radioactive pharmaceutical composition are shown in Table 5.

**[Table 5]**

| Example | name of antibody used | radioactivity (MBq) of reaction solution passed through column | radiochemical purity (%) before purification | after purification | | |
|---|---|---|---|---|---|---|
| | | | | radioactivity (MBq) of recovered eluate | radiochemical yield (%) | radiochemical purity (%) |
| 2 | trastuzumab | 18.0 | 83.0 | 8.2 | 54.7 | 94.9 |
| 3 | panitumumab | 17.3 | 87.4 | 12.0 | 79.4 | 95.8 |
| 4 | rituximab | 17.5 | 85.9 | 7.0 | 46.3 | 95.6 |
| 5 | bevacizumab | 17.5 | 74.7 | 5.8 | 44.5 | 96.5 |

### Example 6: Production of radioactive pharmaceutical composition with ²²⁵Ac-labeled panitumumab as active ingredient

### (1) Complex formation step

The chelating moiety (DOTA-GA-DBCO) similar to that in Example 1 was dispersed in 156 mmol/L acetic acid-sodium acetate buffer (pH 5.5) to obtain a dispersion containing 0.3 mmol/L of the chelating moiety (²²⁵Ac chelating moiety dispersion). This ²²⁵Ac dispersion (139.7 µL) and ²²⁵Ac ion-containing solution (dissolved in 0.1 mol/L hydrochloric acid, radioactivity concentration 192 MBq/mL, 13.415 MBq) (69.9 µL) were added to a 1.5 mL Eppendorf tube (manufactured by Eppendorf, Protein LoBind Tube), and they were reacted under heating conditions to obtain an ²²⁵Ac complex solution. The molar ratio of the chelating moiety to the radioactive metal ion was chelating moiety:²²⁵Ac ion = about 1521:1, the heating temperature of the reaction mixture was 70°C, and the heating time was 30 min.

The radiochemical purity of the ²²⁵Ac complex in the ²²⁵Ac complex solution was measured by TLC analysis, and ²²⁵Ac complex was obtained with a radiochemical purity of 97.3%. The TLC analysis was performed in the same manner as in Example 1 (1), except that a radio γ-TLC analyzer (manufactured by Raytest, Model: GITA Star) was used as the detector.

### (2) Labeling step

The solution of the ²²⁵Ac complex obtained through the aforementioned step (1) and a solution containing the peptide-modified panitumumab (monovalent antibody) produced through Production Example 1 were mixed with His5.5 solvent-substituted solution (799 µL), and a click reaction was performed at 37°C for 2.0 hr to obtain ²²⁵Ac-labeled panitumumab. The radiochemical purity was measured in the same manner as in step (1) except that an acetonitrile:0.1 mol/L EDTA solution (volume ratio 1:1) was used as the developing solvent, and the percentage of the radioactivity (counts) of the peak detected near the point of origin relative to the total radioactivity (counts) detected was used as the radiochemical purity (%), and it was found that ²²⁵Ac-labeled panitumumab was obtained with a radiochemical purity of 60.3%.

### (3) Purification step

The purification column was equilibrated using the same column and procedure as in Example 1(3).

A solution (986.6 µL) containing ²²⁵Ac-labeled panitumumab obtained in step (2) was passed through the above-mentioned equilibrated column, and the ²²⁵Ac-labeled panitumumab was retained in the column. Thereafter, 5 mL of His5.5 was passed through the column twice for washing. Then, 3 mL of RH6.1 was passed through, and the eluent (6.678 MBq) was collected. The radiochemical purity of the eluent was analyzed by TLC in the same manner as in step (2), and the radioactivity of each was measured and the radiochemical yield of the target ²²⁵Ac-labeled panitumumab was calculated. As a result, the radiochemical yield was 49.78%, the recovery rate was 82.55%, and the radiochemical purity was 99.9%. The radiochemical yield is a value expressed as a percentage, which is obtained by dividing the radioactivity of the ²²⁵Ac-labeled antibody contained in 3 mL of the eluent by the radioactivity of the ²²⁵Ac used in the reaction. The recovery rate is a value expressed as a percentage obtained by dividing the radioactivity of the ²²⁵Ac-labeled antibody contained in 3 mL of eluent by a value obtained by multiplying the radioactivity of the ²²⁵Ac used in the reaction by the radiochemical purity in the labeling step (radioactivity of the ²²⁵Ac-labeled antibody).

### (4) Formulation step

The eluate obtained in step (3) was dispensed into three tubes at 0.9 mL each, and each was formulated as entries 1 to 3 using the following procedure.

For entry 1, the dispensed solution (radioactivity concentration 2.23 MBq/mL) was stored as was as a preparation. For entry 2, RH6.1 was added to the dispensed solution to dilute same to a radioactivity concentration of 1.71 MBq/mL, which was then stored as a preparation. For entry 3, the dispensed solution was concentrated by ultrafiltration using a filter (manufactured by Merck, model number: UFC505096), after which RH6.1 was added, and the solution was diluted to a radioactivity concentration of 4.00 MBq/mL and then stored as a preparation.

### Comparative Example 2: Production of radioactive pharmaceutical composition with ²²⁵Ac-labeled panitumumab as active ingredient

### (1) Complex formation step

Performed in the same manner as in Example 6, step (1). The radioactivity of the ²²⁵Ac ion-containing solution used for the reaction was 13.584 MBq.

### (2) Labeling step

Performed in the same manner as in Example 6, step (2). The radiochemical purity was measured in the same manner as in step (1) except that an acetonitrile:0.1 mol/L EDTA solution (volume ratio 1:1) was used as the developing solvent, and the percentage of the radioactivity (counts) of the peak detected near the point of origin relative to the total radioactivity (counts) detected was used as the radiochemical purity (%), and it was found that ²²⁵Ac-labeled panitumumab was obtained with a radiochemical purity of 74.8%.

### (3) Purification step

A purification column was equilibrated with 5 mL of His5.5, 5 mL of NaMES5.5, and 10 mL of His5.5 in this order, and in the same manner as in Example 6 except that 3 mL of NaMES5.5 was used as an eluent, ²²⁵Ac-labeled panitumumab was purified. As a result, an eluent (5.132 MBq) was collected. The radiochemical purity of the eluent was analyzed by TLC in the same manner as in step (2), and the radioactivity of each was measured and the radiochemical yield of the target ²²⁵Ac-labeled panitumumab was calculated. As a result, the radiochemical yield was 37.78%, the recovery rate was 50.51%, and the radiochemical purity was 97.2%. The radiochemical yield is a value expressed as a percentage, which is obtained by dividing the radioactivity of the ²²⁵Ac-labeled antibody contained in 3 mL of the eluent by the radioactivity of the ²²⁵Ac used in the reaction. The recovery rate is a value expressed as a percentage obtained by dividing the radioactivity of the ²²⁵Ac-labeled antibody contained in 3 mL of eluent by a value obtained by multiplying the radioactivity of the ²²⁵Ac used in the reaction by the radiochemical purity in the labeling step (radioactivity of the ²²⁵Ac-labeled antibody).

### (4) Formulation step

The eluate obtained in step (3) was dispensed into three tubes at 0.9 mL each, and each was formulated as entries 4 to 6 using the following procedure.

For entry 4, the dispensed solution was concentrated by ultrafiltration using a filter (manufactured by Merck, model number: UFC505096), after which NaMES5.5 was added, and the solution was diluted to a radioactivity concentration of 2.23 MBq/mL and then stored as a preparation. For entry 5, the dispensed solution (radioactivity concentration 1.71 MBq/mL) was stored as was as a preparation. For entry 6, the dispensed solution was concentrated by ultrafiltration using a filter (manufactured by Merck, model number: UFC505096), after which NaMES5.5 was added, and the solution was diluted to a radioactivity concentration of 4.00 MBq/mL and then stored as a preparation.

### Experimental Example 1: Evaluation of influence of eluent composition on storage stability of formulation

The storage stability of the radioactive pharmaceutical compositions (radioactive preparations) of entries 1 to 6 prepared in Example 6 and Comparative Example 2 was verified when stored at room temperature from immediately after production until 14 days later. In addition, the storage stability was also verified when stored at 37°C from 10 days onwards after production. The results thereof are shown in Table 6.

**[Table 6]**

| lot | composition | radioactivity concentration (MBq/mL) | radiochemical purity (%) immediately after production | decrease in radiochemical purity (%) from immediately after production | | | |
|---|---|---|---|---|---|---|---|
| | | | | 7 days | 10 days | 14 days | |
| | | | | room temperature | room temperature | room temperature | 37°C |
| entry 1 | RH6.1 | 2.23 | 99.9 | 0.8 | 3.6 | 3.3 | 5.5 |
| entry 2 | RH6.1 | 1.71 | 98.3 | 0.9 | 1.2 | 3.4 | 4.2 |
| entry 3 | RH6.1 | 4.00 | 98.1 | 0.7 | 2.0 | 2.8 | 5.9 |
| entry 4 | NaMES5.5 | 2.23 | 98.5 | 2.9 | 7.2 | 10.9 | 14.5 |
| entry 5 | NaMES5.5 | 1.71 | 97.2 | 1.5 | 4.3 | 10.8 | 25.2 |
| entry 6 | NaMES5.5 | 4.00 | 97.8 | 4.9 | 7.1 | 13.3 | 25.1 |

From the results in Table 6, it was shown that entries 1 to 3 containing RH6.1 have the effect of suppressing the decrease in radiochemical purity over time at all radioactivity concentrations, compared to entries 4 to 6 containing NaMES5.5. A similar tendency was observed in storage at 37°C after 10 days, and entries 1 to 3 had the effect of suppressing a decrease in radiochemical purity over time, compared to entries 4 to 6.

This application is based on a patent application No. 2022-171833 filed in Japan (filing date: October 26, 2022), the contents of which are incorporated in full herein.

## Claims

1. A method for producing a liquid radioactive pharmaceutical composition containing, as an active ingredient, a radiolabeled antibody obtained by labeling an antibody with a radionuclide, and containing one or more additives (A), comprising:
(Step 1) a step of labeling an antibody with a radionuclide to obtain a solution of a crude product of the radiolabeled antibody;
(Step 2) a step of contacting the solution of the crude product obtained in the Step 1 with a cation exchange material to adsorb the radiolabeled antibody onto the cation exchange material; and
(Step 3) a step of eluting the radiolabeled antibody adsorbed onto the cation exchange material in the Step 2 with an eluent containing the additive (A).

2. The method according to claim 1, wherein the radioactive pharmaceutical composition contains a second additive (B) in addition to the first additive which is the additive (A), and a step of adding a diluent containing the second additive (B) to the eluate containing the radiolabeled antibody is performed after practicing Step 3.

3. The method according to claim 1 or 2, wherein the radionuclide is a radioactive metal nuclide or a radioactive halogen nuclide.

4. The method according to claim 1 or 2, wherein the radionuclide is Al¹⁸F, ⁸⁹Zr, ⁹⁰Y, ¹¹¹In, ¹⁷⁷Lu, or ²²⁵Ac.

5. The method according to claim 1 or 2, wherein the cation exchange material has a sulfopropyl group or a carboxymethyl group as an electric charge group.

6. The method according to claim 1 or 2, wherein the cation exchange material is packed in a column.

7. The method according to claim 1 or 2, wherein a step of washing the cation exchange material by passing a washing solution through the cation exchange material onto which the radiolabeled antibody is adsorbed is performed before practicing the Step 3.

8. The method according to claim 7, wherein the washing solution contains one or more pH adjusters.

9. The method according to claim 8, wherein the pH adjuster is at least one buffer selected from the group consisting of histidine, 2-morpholinoethanesulfonic acid (MES), 2-[bis(2-hydroxyethyl)amino]-2-(hydroxymethyl)propane-1,3-diol (Bis-Tris), N-(2-acetamido)iminodiacetic acid (ADA), piperazine-1,4-bis(2-ethanesulfonic acid) (PIPES), N-(2-acetamido)-2-aminoethanesulfonic acid (ACES), 2-hydroxy-3-morpholinopropanesulfonic acid (MOPSO), N,N-bis(2-hydroxyethyl)-2-aminoethanesulfonic acid (BES), 3-morpholinopropane-1-sulfonic acid (MOPS), N-tris (hydroxymethyl)methyl-2-aminoethanesulfonic acid (TES), and 2-[4-(2-hydroxyethyl)-1-piperazinyl]ethanesulfonic acid (HEPES).

10. The method according to claim 7, wherein the washing solution has a pH of 4 to 7.

11. The method according to claim 1 or 2, wherein the radioactive pharmaceutical composition comprises a positively-charged additive (a1) and a pH adjuster as the additive (A), and
the eluent comprises the positively-charged additive (a1) and the pH adjuster contained in the radioactive pharmaceutical composition.

12. The method according to claim 11, wherein the positively-charged additive (a1) comprises a compound having a guanidine skeleton.

13. The method according to claim 12, wherein the compound having a guanidine skeleton is at least one compound selected from the group consisting of arginine, acylated arginine, and agmatine.

14. The method according to claim 12, wherein the positively-charged additive (a1) further comprises at least one compound selected from the group consisting of sodium chloride, potassium chloride, and sodium acetate.

15. The method according to claim 11, wherein the pH adjuster comprises at least one buffering agent selected from the group consisting of histidine, 2-[4-(2-hydroxyethyl)-1-piperazinyl]ethanesulfonic acid (HEPES), phosphoric acid, and citric acid.

16. The method according to claim 1 or 2, wherein the eluent containing the additive (A) comprises a histidine buffer containing arginine.

17. The method according to claim 16, wherein the eluent comprises 10 - 500 mmol/L of arginine and 10 - 300 mmol/L of histidine.

18. The method according to claim 1 or 2, wherein the eluent has 6<pH≤8.

19. A method for purifying a radiolabeled antibody, comprising:
(Step A) a step of labeling an antibody with a radionuclide to obtain a solution of a crude product of a radiolabeled antibody;
(Step B) a step of contacting the solution of the crude product obtained in the Step A with a cation exchange material to adsorb the radiolabeled antibody onto the cation exchange material; and
(Step C) a step of eluting the radiolabeled antibody adsorbed onto the cation exchange material in the Step B with an eluent containing one or more additives.
